(19) Europäisches Patentamt / European Patent Office / Office européen des brevets

(11) **EP 4 018 004 B1**

(12) **EUROPEAN PATENT SPECIFICATION**

(45) Date of publication and mention of the grant of the patent:
**18.06.2025 Bulletin 2025/25**

(21) Application number: **20761997.4**

(22) Date of filing: **14.08.2020**

(51) International Patent Classification (IPC):
**C12Q 1/689** (2018.01)

(52) Cooperative Patent Classification (CPC):
**C12Q 1/689**

(86) International application number:
**PCT/US2020/046406**

(87) International publication number:
**WO 2021/041056 (04.03.2021 Gazette 2021/09)**

(54) **COMPOSITIONS, METHODS AND KITS FOR DETECTING TREPONEMA PALLIDUM**

ZUSAMMENSETZUNGEN, VERFAHREN UND KITS ZUM NACHWEIS VON TREPONEMA PALLIDUM

COMPOSITIONS, PROCÉDÉS ET KITS POUR LA DÉTECTION DE TREPONEMA PALLIDUM

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **23.08.2019 US 201962891181 P**

(43) Date of publication of application:
**29.06.2022 Bulletin 2022/26**

(73) Proprietor: **GEN-PROBE INCORPORATED**
San Diego, CA 92121 (US)

(72) Inventors:
• O'DONNELL, Meghan, A.
San Diego, CA 92109 (US)
• DARBY, Paul, M.
San Diego, CA 92126 (US)
• GETMAN, Damon, K.
San Diego, CA 92064 (US)

(74) Representative: **Script Intellectual Property LLP**
Suite J, Anchor House
School Close
Chandlers Ford
Eastleigh, Hampshire SO53 4DY (GB)

(56) References cited:
WO-A1-2006/025672    WO-A2-00/01850

• MATTHEW GOLDEN ET AL: "Treponema pallidum Nucleic Acid Amplification Testing To Augment Syphilis Screening among Men Who Have Sex with Men", JOURNAL OF CLINICAL MICROBIOLOGY, vol. 57, no. 8, 12 June 2019 (2019-06-12), US, pages 1 - 7, XP055737822, ISSN: 0095-1137, DOI: 10.1128/JCM.00572-19
• CENTURION-LARA A ET AL: "Detection of Treponema pallidum by a Sensitive Reverse Transcriptase PCR", JOURNAL OF CLINICAL MICROBIOLOGY, AMERICAN SOCIETY FOR MICROBIOLOGY, US, vol. 35, no. 6, 1 June 1997 (1997-06-01), pages 1348 - 1352, XP002999607, ISSN: 0095-1137
• DESY A. GULTOM ET AL: "Detection and identification of azithromycin resistance mutations on <em>Treponema pallidum</em> 23S rRNA gene by nested multiplex polymerase chain reaction", MEDICAL JOURNAL OF INDONESIA, vol. 26, no. 2, 18 August 2017 (2017-08-18), pages 90 - 6, XP055737825, ISSN: 0853-1773, DOI: 10.13181/mji.v26i2.1543
• KENNETH A. BROWNE: "Sequence-Specific, Self-Reporting Hairpin Inversion Probes", JOURNAL OF THE AMERICAN CHEMICAL SOCIETY, vol. 127, no. 6, 1 February 2005 (2005-02-01), pages 1989 - 1994, XP055047379, ISSN: 0002-7863, DOI: 10.1021/ja046369w

Remarks:

The complete document including Reference Table(s) and the Sequence Listing(s) can be downloaded from the EPO website

Remarks:

The complete document including Reference Table(s) and the Sequence Listing(s) can be downloaded from the EPO website

**Description**

**Related Applications**

This application claims the benefit of U.S. Provisional Application No. 62/891,181, filed August 23, 2019.

**Technical Field**

The present disclosure relates to the field of biotechnology. More specifically, the disclosure concerns nucleic acid diagnostics. Still more specifically, the disclosure relates to an assay for detecting the nucleic acid of *T. pallidum,* the infectious agent that causes syphilis.

**Background**

Syphilis is a sexually transmitted infection caused by the spirochete *Treponema pallidum.* This motile bacterium also can be transmitted to a fetus by transplacental passage during the later stages of pregnancy, giving rise to congenital syphilis. Asymptomatic incubation can last for 3 to 90 days (21 days on average), leaving a significant window period between exposure and detectable infection. (Henao-Martinez et al., Nuerol Clin Pract. 4(2):114-122 (2014))

Traditional diagnostic testing has relied on the combination of clinical findings and serologic assays. This can, for example, involve detection of antibodies directed against *T. pallidum* proteins. Different procedures may involve dark-field microscopy, PCR, and direct fluorescent antibody testing for *T. pallidum.* While few clinical testing centers have these tools available, it is possible to obtain a diagnosis of syphilis prior to serologic conversion. (Henao-Martinez *et al.,* (2014))

An analysis of the complete genomic sequence of *T. pallidum* has been published by Fraser et. al., in Science 281:375-388 (1998). Interestingly, the entire genonic consists of only about 1.14 Mbp, and contains 1041 open reading frames. This compact genome size likely reflects the dependence of the organism on many functions provided by its mammalian host.

WO 2006/025672, Golden M. et al. J Clin Mircobiol (2029) 57(8):1-7, Centurion-Lara A. et al. J Clin Microbiol (1997) 35(6):1348-1352, and Gultom, D. A. et al. Med J Indonesia (2017) 26(2):90-96 disclose primers and probes for detecting *T. pallidum* and methods using these for detecting *T. pallidum.*

Given that clinical manifestations of syphilis can vary widely and can sometimes easily be confused with other infections, and that false-positive non-treponemal tests have been reported for other conditions (including pregnancy), there is a need for a sensitive assay that is specific for *T. pallidum.*

**Summary of the Disclosure**

The aspects and embodiments of the present invention are set forth in the claims.

**Brief Description of the Drawings**

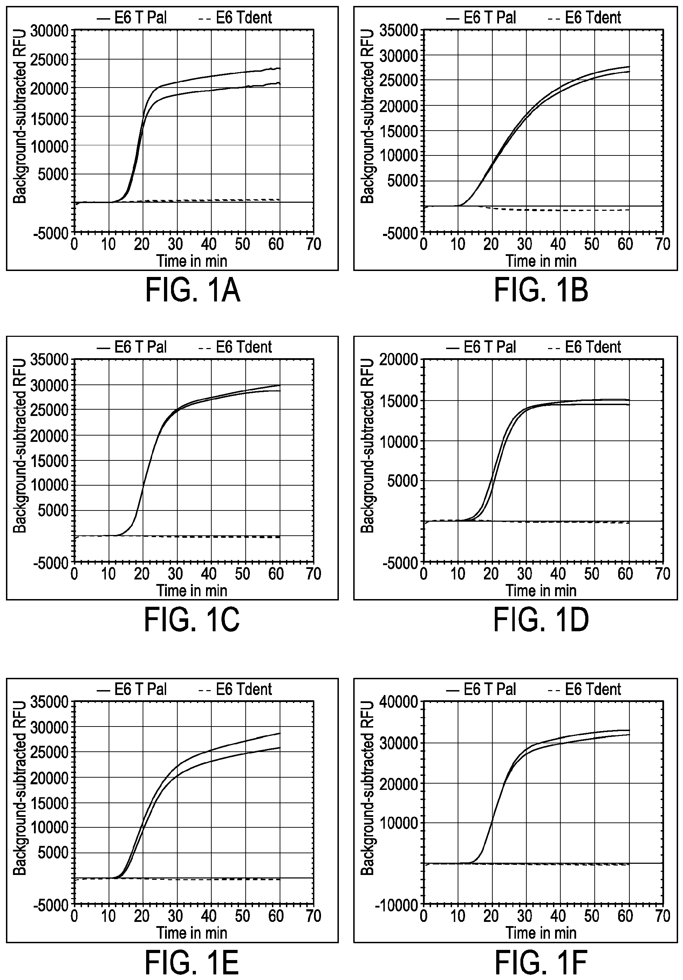

Figures 1A-1F are graphical real-time run curves presenting background subtracted fluorescence (y-axis) as a function of reaction time (x-axis). Figure 1A shows results for system S2. Figure 1B shows results for system S3. Figure 1C shows results for system S4.

Figure 1D shows results for system S5. Figure 1E shows results for system S6. Figure 1F shows results for system S7. In all instances, "Tpal" refers to results obtained using the *T. pallidum* target nucleic acid, while "Tdent" refers to results obtained using the *T. denticola* target nucleic acid.

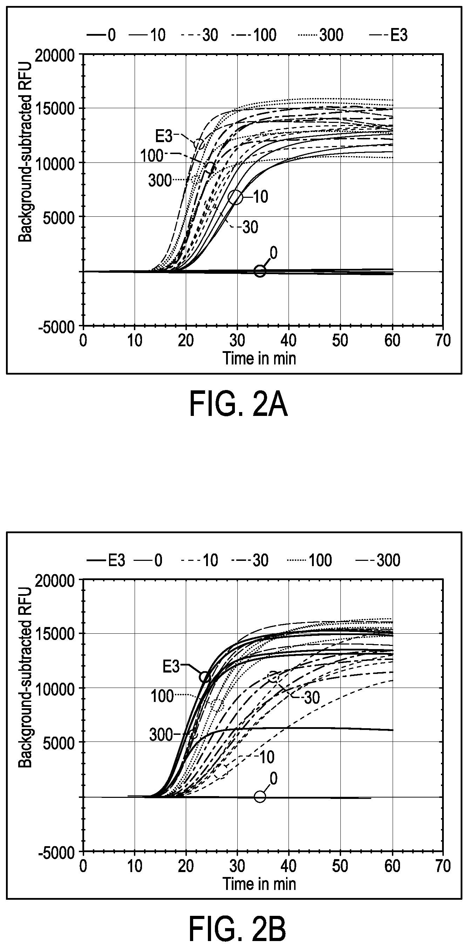

Figures 2A-2B are graphical real-time run curves presenting background subtracted fluorescence (y-axis) as a function of reaction time (x-axis). Figure 2A shows results for biphasic amplification using oligonucleotides of system S5. Figure 2B shows results for biphasic amplification using oligonucleotides of system S7.

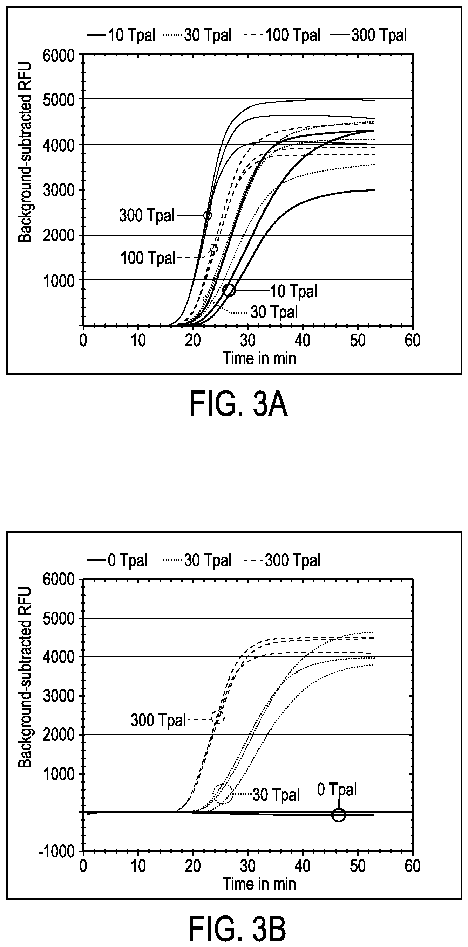

Figures 3A-3B are graphical real-time run curves presenting background subtracted fluorescence (y-axis) as a function of reaction time (x-axis). Figure 3A illustrates sensitivity of the *T. pallidum* detection assay. Figure 3B illustrates that detection of *T. pallidum* nucleic acid was highly specific. In all instances, "Tpal" refers to results obtained using the *T. pallidum* target nucleic acid. Introduction and Overview

The present disclosure provides a solution to the clinical need for a sensitive assay that is specific for *T. pallidum.* Featured are methods useful for determining whether *T. pallidum* is present in a test sample obtained from, for instance, a swab taken from an individual being tested for this bacterium. Specimens for determining the presence of *T. pallidum* may be obtained from the genital tract, cultures, bacterial lysates, and other sample media.

The present disclosure also provides a solution to the clinical need for an assay specific for *T. pallidum* by featuring oligonucleotides useful for determining whether *T. pallidum* is present in a test sample obtained from, for example, the urethra, the anal canal, the respiratory tract, or the lower genital tract of a human. The featured oligonucleotides may be hybridization assay probes, capture probes and/or amplification primers for detecting, immobilizing and/or amplifying target nucleic acid sequences derived from *T. pallidum* in a test sample. Particularly disclosed are target regions for detecting *T. pallidum* in sequences derived from the 23S rRNA or the complement thereof.

In one embodiment of the present disclosure, hybridization assay probes are provided which hybridize to a 23S rRNA target region present in nucleic acid derived from *T. pallidum,* or the complement thereof, to form detectable probe:target hybrids that indicate the presence of *T. pallidum* in a test sample. Primers and probes of this embodiment include oligonucleotides having target binding regions, where the base sequences of the target binding regions are contained within the base sequence of (SEQ ID NO: 1) or the complement thereof, allowing for RNA and DNA equivalent base substitutions, and backbone modifications or analogs.

Oligonucleotide hybridization probes (or simply "probes") can be used to preferentially detect the *T. pallidum* target nucleic acid over nucleic acid derived from non-*T. pallidum* organisms, especially over nucleic acid derived from *T. denticola,* for example under stringent hybridization assay conditions.

Target binding regions of preferred probes may include DNA, RNA, a combination DNA and RNA, or may include a nucleic acid analog (*e.g.,* a peptide nucleic acid) or one or more modified nucleosides (*e.g.,* a ribonucleoside having a ribofuranosyl moiety with a 2'-O-methyl substitution). Probes of the present disclosure are preferably oligonucleotides of at least 13 bases, and up to 22, 25, 50 or even 100 bases in length. Most preferably, hybridization probes of the present disclosure are nucleic acids or nucleic acid analogs consisting of the recited sequence, and optionally include a detectable label or reporter group.

While not required, the probes can include either a detectable label or a group of interacting labels. The label may be any suitable labeling substance, including but not limited to a radioisotope, an enzyme, an enzyme cofactor, an enzyme substrate, a dye, a hapten, a chemiluminescent molecule, a fluorescent molecule, a phosphorescent molecule, an electrochemiluminescent molecule, a chromophore, a base sequence region that is unable to stably bind to the target nucleic acid under the stated conditions, and mixtures of these. Groups of interacting labels that can be used include, but are not limited to, enzyme/substrate, enzyme/cofactor, fluorophore/quencher, luminescent/adduct, dye dimers and Förrester energy transfer pairs.

In some embodiments, probes harbor homogeneous detectable labels, such as chemiluminescent labels that can be detected in a homogeneous assay format. Examples of preferred chemiluminescent labels include acridinium ester labels.

Hybridization assay probes of the present disclosure optionally can include, in addition to the base sequence of the target binding region, one or more base sequences which do not stably bind to nucleic acid derived from the target organism (*i.e., T. pallidum*) under stringent conditions. By way of example, an additional base sequence may constitute the immobilized probe binding region of a capture probe, where the immobilized probe binding region is comprised of, for example, a 3' poly(dA) region which hybridizes under stringent conditions to a 5' poly(dT) region of a polynucleotide bound directly or indirectly to a solid support. An additional base sequence might also be a 5' sequence recognized by an RNA polymerase or which enhances initiation or elongation by an RNA polymerase (*e.g.,* a T7 promoter). More than one additional base sequence may be included if the first sequence is incorporated into, for example, a "molecular beacon" probe. Molecular beacons are disclosed by Tyagi et al., "Detectably Labeled Dual Conformation Oligonucleotide Probes, Assays and Kits," U.S. Patent No. 5,925,517, and include a target binding region which is bounded by two base sequences having regions which are at least partially complementary to each other. The "molecular torch" is yet another example of a dual-labeled structured hybridization probe that can be used to detect *T. pallidum* nucleic acid sequences or amplification products indicative of the presence of *T. pallidum.* As discussed elsewhere herein, molecular torch hybridization probes have been disclosed by Becker et al., in U.S. Patent No. 6,361,945. Molecular torches can include an additional base sequence joined directly to the target binding region or, for example, by means of a non-nucleotide (*e.g.,* C9) linker.

Also contemplated are compositions comprising stable nucleic acid duplexes formed between one or more oligonucleotides and nucleic acid amplification products under conditions permitting nucleic acid hybridization, or nucleic acid amplification. For example, there can be an RNA amplification product having polarity opposite the naturally occurring rRNA template or target, and the amplification product can be hybridized to a synthetic oligonucleotide (*e.g.,* a primer or a detectably labeled hybridization probe). In some embodiments, the hybridization probe can be a dual-labeled hybridization probe that includes a fluorophore and a quencher. Such probes include molecular beacons, molecular torches, and hydrolysis probes.

In another embodiment, the disclosure contemplates probe mixes that are useful for determining whether *T. pallidum* organisms are present in a test sample. For instance, to determine the presence of these organisms, the probe mix can include one or more of the *T. pallidum* probes described herein.

In a further embodiment, the present disclosure provides capture probes including at least one oligonucleotide containing an immobilized probe binding region and a target binding region. The immobilized probe binding region of the capture probes can include any base sequence capable of stably hybridizing under stringent conditions to oligonucleotides bound to a solid support present in a test sample. Preferably, the immobilized probe binding region is a poly (dA), homopolymer tail located at the 3' end of the capture probe. In this embodiment, oligonucleotides bound to the solid support would include 5' poly (dT) tails of sufficient length to stably bind to the poly (dA) tails of the capture probes under assay conditions. In a preferred embodiment, the immobilized probe binding region includes a poly (dA) tail which is about 30 adenines in length, and the capture probe includes a spacer region which is about 3 thymines in length for joining target binding region and the immobilized probe binding region to each other.

The disclosure also features amplification primers useful for detecting the presence of *T. pallidum* nucleic acids in an amplification assay. In one preferred embodiment, each amplification primer includes an oligonucleotide, where the base sequence of the target binding region of the primer has or substantially corresponds to a base sequence contained within: SEQ ID NO:3, SEQ ID NO:4, or SEQ ID NO:5. Particular examples of target-complementary oligonucleotide sequences used for priming nucleic acid synthesis are given by SEQ ID NO:6, SEQ ID NO:7, and SEQ ID NO:8. Amplification primers of the present disclosure optionally include a 5' promoter sequence which is recognized by an RNA polymerase or which enhances initiation or elongation by an RNA polymerase. If included, a T7 promoter, such as AA TTTAATACGACTCACTATAGGGAGA (SEQ ID NO:9) is preferred. Particular examples of T7 promoter primers disclosed herein include: SEQ ID NO:10, SEQ ID NO:11, and SEQ ID NO:12.

Amplification primers of the present disclosure are preferably employed in sets of at least two amplification primers. Preferred sets include a first amplification primer that includes an oligonucleotide having a target binding region, where the base sequence of the target binding region contains an at least 18 contiguous base region which is at least about 80% complementary (more preferably at least about 90% complementary and most preferably 100% complementary) to an at least 18 contiguous base region present in a target sequence selected from the group consisting of SEQ ID NO:3, SEQ ID NO:4, and SEQ ID NO:5. Optionally, the first amplification primer includes a promoter sequence attached upstream of the target-hybridizing sequence *(e.g.,* at its 5' end). The second amplification primer of these preferred sets comprises an oligonucleotide having a target binding region, where the base sequence of the target binding region contains an at least 17 contiguous base region which is at least about 80% complementary (more preferably at least about 90% complementary and most preferably 100% complementary) to an at least 17 contiguous base region present in a target sequence selected from the group consisting of SEQ ID NO:13, SEQ ID NO:14, and SEQ ID NO:15.

Nucleic acid amplification products (*i.e.,* "amplicons") synthesized by the combined use of the first and second amplification primers can be detected by the use of labeled hybridization probes. In certain embodiments, hybridization probes include a sequence of at least 13, 14, 16, or 22 contiguous bases of the sequence of any of SEQ ID NO:19, SEQ ID NO:20, and SEQ ID NO:21, each allowing for complements thereof and RNA and DNA equivalent base substitutions.

The disclosure further features methods for determining whether *T. pallidum* is present in a test sample. In one embodiment, the method includes the steps of: (a) contacting the test sample with one of the above-described hybridization assay probes for detecting *T. pallidum* under conditions permitting the probe to preferentially hybridize to a target nucleic acid derived from *T. pallidum* (*e.g.,* including nucleic acid amplification products), thereby forming a probe: target hybrid stable for detection; and (b) determining whether the hybrid is present in the test sample as an indication of the presence or absence of *T. pallidum* in the test sample. This method may further include the step of quantifying the amount of hybrid present in the test sample as a means for estimating the amount of *T. pallidum* present in the test sample. Preferably, the probe includes a detectable label, where the label produces a detectable signal indicating the probe is hybridized to nucleic acids derived from *T. pallidum* rather than another species. For example, the signal can be at least two times, at least five times, at least ten times, or at least five hundred times stronger when the probe is hybridized to nucleic acids derived from *T. pallidum* compared to when the probe is hybridized to nucleic acids derived from *T. denticola.*

The disclosure also features methods for amplifying a target nucleic acid sequence present in nucleic acid derived from *T. pallidum* present in a test sample, where the method includes the steps of: (a) contacting the test sample with at least one of the above-described amplification primers under amplification conditions; and (b) amplifying the target nucleic acid sequence. Preferred amplification methods will include a set of at least two of the above-described amplification primers.

In one embodiment, the method for amplifying a target nucleic acid sequence present in nucleic acid derived from *T. pallidum* will further include the steps of: (a) contacting the test sample with a hybridization assay probe which preferentially hybridizes to the target nucleic acid sequence, or a complement thereof, under stringent hybridization conditions, thereby forming a probe:target hybrid stable for detection; and (b) determining whether the hybrid is present in the test sample as an indication of the presence or absence of *T. pallidum* in the test sample. This can involve determining whether or not a nucleic acid amplification product is synthesized using a primer in accordance with the disclosure. The above-described hybridization assay probes are especially preferred for this method.

The disclosure also features reaction mixtures that can be used for detecting nucleic acids of *T. pallidum.* These reaction mixtures may include the sample undergoing testing, a set of primers, and a detectably labeled hybridization probe. The primers and probes have sequences in accordance with the disclosure and claims, herein.

The disclosure also features kits for amplifying a target nucleic acid sequence present in nucleic acid derived from *T. pallidum,* where the kits include at least one of the above-described amplification primers and one or more hybridization probes for detecting nucleic acids of *T. pallidum.* In a further embodiment, these kits may include, in addition to the amplification primers and hybridization probes, at least one of the above-described capture probes. Such kits may further include a solid support material for immobilizing the capture probe in a test sample.

## Detailed Description

Disclosed herein are oligonucleotides targeted to nucleic acid derived from *T. pallidum,* where the oligonucleotides are useful for determining the presence or absence of *T. pallidum* in a test sample. The oligonucleotides can aid in detecting *T. pallidum* in different ways, such as by functioning as hybridization assay probes, capture probes and/or amplification primers. Hybridization assay probes of the present disclosure can preferentially hybridize to a target nucleic acid sequence present in nucleic acid derived from *T. pallidum* under stringent hybridization assay conditions to form detectable duplexes which indicate the presence of *T. pallidum* in a test sample. Preferred probes are capable of distinguishing between the target organism and its nearest known phylogenetic neighbors. Capture probes of the present disclosure can hybridize to a target nucleic acid sequence present in nucleic acid derived from *T. pallidum* under stringent hybridization assay conditions and can be used to separate target nucleic acid from clinical specimens. Amplification primers of the present disclosure can hybridize to a target nucleic acid sequence present in nucleic acid derived from *T. pallidum* under amplification conditions, and can be used as primers in an amplification reaction to generate *T. pallidum-derived* nucleic acid. The probes and amplification primers may be used in assays for detecting and/or quantifying *T. pallidum* in a test sample.

Those skilled in the art will appreciate that the hybridization assay probes of the present disclosure may be used as amplification primers or capture probes; that the target binding regions of the amplification primers of the present disclosure may be used as hybridization assay probes or capture probes, depending upon the degree of specificity required by a particular assay; and that the target binding regions of the capture probes of the present disclosure may be used as hybridization assay probes or amplification primers, depending upon the degree of specificity required by a particular assay. Thus, the present disclosure contemplates oligonucleotides for use in determining the presence or absence of *T. pallidum* in a test sample comprising, consisting essentially of, or consisting of any of the nucleotide base sequences disclosed herein, optionally including one or more nucleotide analogs.

## Definitions

The following terms have the indicated meanings in the specification unless expressly indicated to have a different meaning.

The terms "a," "an," and "the" include plural referents, unless the context clearly indicates otherwise. For example, "a nucleic acid" as used herein is understood to represent one or more nucleic acids. As such, the terms "a" (or "an"), "one or more," and "at least one" can be used interchangeably herein.

By "sample" or "test sample" is meant any substance suspected of containing a target organism or nucleic acid derived from the target organism. The substance may be, for example, an unprocessed clinical specimen, such as a sputum or urethral specimen, a buffered medium containing the specimen, a medium containing the specimen and lytic agents for releasing nucleic acid belonging to the target organism, or a medium containing nucleic acid derived from the target organism which has been isolated and/or purified in a reaction receptacle or on a reaction material or device. As used herein, the terms "sample" and "test sample" may refer to specimen in its raw form or to any stage of processing to release, isolate and purify nucleic acid derived from target organisms in the specimen.

A "nucleotide" as used herein is a subunit of a nucleic acid consisting of a phosphate group, a 5-carbon sugar, and a nitrogenous base (also referred to herein as "nucleobase"). The 5-carbon sugar found in RNA is ribose. In DNA, the 5-carbon sugar is 2'-deoxyribose. The term also includes analogs of such subunits, such as a methoxy group at the 2' position of the ribose (also referred to herein alternatively as "2'-O-methyl" or "2'-O-Me" or "2'-methoxy" or "2'-OMe").

"Analog" refers to two or more chemical compounds, where the compounds exhibit a similar or related chemical architecture. Despite exhibiting shared structural similarities, each analog may have very different biochemical properties.

"Nucleic acid" and "polynucleotide" refer to a multimeric compound comprising nucleotides or nucleotide analogs having nitrogenous heterocyclic bases or base analogs linked together to form a polynucleotide, including conventional RNA, DNA, mixed RNA-DNA, and polymers that are analogs thereof. A nucleic acid "backbone" may be made up of a variety of linkages, including one or more of sugar-phosphodiester linkages, peptide-nucleic acid bonds ("peptide nucleic acids" or PNA; PCT Publication No. WO 95/32305), phosphorothioate linkages, methylphosphonate linkages, or combinations thereof. Sugar moieties of a nucleic acid may be ribose, deoxyribose, or similar compounds with substitutions (*e.g.*, 2' methoxy or 2' halide substitutions). Nitrogenous bases may be conventional bases (A, G, C, T, U), analogs thereof (*e.g.,* inosine or others; see The Biochemistry of the Nucleic Acids 5-36, Adams et al., ed., 11th ed., 1992), derivatives of purines or pyrimidines (*e.g.,* $N^4$-methyl deoxyguanosine, deaza- or aza-purines, deaza- or aza-pyrimidines, pyrimidine bases with substituent groups at the 5 or 6 position, purine bases with a substituent at the 2, 6, or 8 positions, 2-amino-6-methylaminopurine, $O^6$-methylguanine, 4-thio-pyrimidines, 4-amino-pyrimidines, 4-dimethylhydrazine-pyrimidines, and $O^4$-alkyl-pyrimidines; U.S. Patent No. 5,378,825 and PCT Publication No. WO 93/13121). Nucleic acids may include one or more "abasic" residues where the backbone includes no nitrogenous base for position(s) of the polymer (U.S. Patent No. 5,585,481). A nucleic acid may comprise only conventional RNA or DNA sugars, bases and linkages, or may include both conventional components and substitutions (*e.g.,* conventional bases with 2' methoxy backbones, or polymers containing both conventional bases and one or more base analogs). Nucleic acid includes "locked nucleic acid" (LNA), an analog containing one or more LNA nucleotide monomers with a bicyclic furanose unit locked in an RNA mimicking sugar conformation, which enhance hybridization affinity toward complementary RNA and DNA sequences (Vester and Wengel, 2004, Biochemistry 43(42):13233-41). Embodiments of oligomers that may affect stability of a hybridization complex include PNA oligomers, oligomers that include 2'-methoxy or 2'-fluoro substituted RNA, or oligomers that affect the overall charge, charge density, or steric associations of a hybridization complex, including oligomers that contain charged linkages (*e.g.,* phosphorothioates) or neutral groups (*e.g.,* methylphosphonates). 5-methylcytosines may be used in conjunction with any of the foregoing backbones/sugars/linkages including RNA or DNA backbones (or mixtures thereof) unless otherwise indicated. It is understood that when referring to ranges for the length of an oligonucleotide, amplicon, or other nucleic acid, that the range is inclusive of all whole numbers (*e.g.,* 19-25 contiguous nucleotides in length includes 19, 20, 21, 22, 23, 24, and 25).

"Oligomer," "oligonucleotide," or "oligo" refers to a nucleic acid of generally less than 1,000 nucleotides (nt), including those in a size range having a lower limit of about 2 to 5 nucleotides and an upper limit of about 500 to 900 nucleotides. Some particular embodiments are oligomers in a size range with a lower limit of about 13, 14, 15, 16, 17, 18, 19, 20, 21, 22, 23, 24, or 25 nucleotides and an upper limit of about 50 to 60 nucleotides, and other particular embodiments are in a size range with a lower limit of about 10 to 20 nucleotides and an upper limit of about 22 to 100 nucleotides. Oligomers may be purified from naturally occurring sources, but may be synthesized by using any well-known enzymatic or chemical method. The term oligonucleotide does not denote any particular function of the reagent; rather, it is used generically to cover all such reagents described herein. An oligonucleotide may serve various different functions. For example, it may function as a primer if it is specific for and capable of hybridizing to a complementary strand and can further be extended in the presence of a nucleic acid polymerase; it may function as a primer and provide a promoter if it contains a sequence recognized by an RNA polymerase and allows for transcription (*e.g.,* a T7 Primer); and it may function to detect a target nucleic acid if it is capable of hybridizing to the target nucleic acid, or an amplicon thereof, and further provides a detectible moiety (*e.g.,* an acridinium-ester compound). Oligomers may be referred to by a functional name (*e.g.,* capture probe, primer or promoter primer) but those skilled in the art will understand that such terms refer to oligomers.

Oligonucleotides of a defined sequence may be produced by techniques known to those of ordinary skill in the art, such as by chemical or biochemical synthesis, and by *in vitro* or *in vivo* expression from recombinant nucleic acid molecules (*e.g.,* bacterial or retroviral vectors). As intended by this disclosure, an oligonucleotide may not consist of wild-type chromosomal DNA or the *in vivo* transcription products thereof. For example, oligonucleotide hybridization probes can include non-nucleotide linkers and/or detectable labels that are not found in naturally occurring nucleic acids.

"Detection probe oligomer," "detection probe," or "probe" refers to an oligomer that hybridizes specifically to a target sequence, including an amplified sequence, under conditions that promote nucleic acid hybridization, for detection of the target nucleic acid. Detection may either be direct (*i.e.,* probe hybridized directly to the target or amplification product thereof) or indirect (*i.e.,* a probe hybridized to an intermediate structure that links the probe to the target or amplification product thereof). Detection probes may be DNA, RNA, analogs thereof or combinations thereof (*e.g.,* DNA/RNA chimerics), and they may be labeled or unlabeled. Detection probes may further include alternative backbone linkages (*e.g.,* 2'-O-methyl linkages). A probe's target sequence generally refers to the specific sequence within a larger sequence to which the probe hybridizes specifically. A detection probe may include target-specific sequence(s) and non-target-specific sequence(s). Such non-target-specific sequences can include sequences which will confer a desired secondary or tertiary structure, such as a hairpin structure, which can be used to facilitate detection and/or amplification (*see, e.g.,* U.S. Patent Nos. 5,118,801, 5,312,728, 6,835,542, and 6,849,412). Probes of a defined sequence may be produced by techniques known to those of ordinary skill in the art, such as by chemical synthesis, and by *in vitro* or *in vivo* expression from recombinant nucleic acid molecules.

"Label" or "detectable label" refers to a moiety or compound joined directly or indirectly to a probe that is detected or leads to a detectable signal. Direct joining may use covalent bonds or noncovalent interactions (*e.g.,* hydrogen bonding, hydrophobic or ionic interactions, and chelate or coordination complex formation) whereas indirect joining may use a bridging moiety or linker (*e.g.,* via an antibody or additional oligonucleotide(s)). Any detectable moiety may be used, including a radionuclide, a ligand such as biotin or avidin or even a polynucleotide sequence, an enzyme, an enzyme substrate, a reactive group, a chromophore such as a dye or particle (*e.g.,* a latex or metal bead) that imparts a detectable color, a luminescent compound (*e.g.,* bioluminescent, phosphorescent, or a chemiluminescent compound), and a fluorescent compound or moiety (*i.e.,* fluorophore). Embodiments of fluorophores include those that absorb light in the range of about 495 to 650 nm and emit light in the range of about 520 to 670 nm, which include those known as FAM™, TET™, CAL FLUOR™ (Orange or Red), and QUASAR™ compounds. Fluorophores may be used in combination with a quencher molecule that absorbs light when in close proximity to the fluorophore to diminish background fluorescence. Such quenchers are well known in the art and include, for example, BLACK HOLE QUENCHER™ (or BHQ™) or TAMRA™ compounds. Particular embodiments include a "homogeneous detectable label" that is detectable in a homogeneous system in which bound labeled probe in a mixture exhibits a detectable change compared to unbound labeled probe, which allows the label to be detected without physically removing hybridized from unhybridized labeled probe (*e.g.,* US Pat. Nos. 5,283,174, 5,656,207, and 5,658,737). Particular homogeneous detectable labels include chemiluminescent compounds, including acridinium ester ("AE") compounds, such as standard AE or AE derivatives which are well known (US Pat. Nos. 5,656,207, 5,658,737, and 5,639,604). Methods of synthesizing labels, attaching labels to nucleic acid, and detecting signals from labels are well known (*e.g.,* Sambrook et al., Molecular Cloning, A Laboratory Manual, 2nd ed. (Cold Spring Harbor Laboratory Press, Cold Spring Harbor, NY, 1989) at Chapt. 10, and US Pat. Nos. 5,658,737, 5,656,207, 5,547,842, 5,283,174, and 4,581,333, and EP Pat. App. 0 747 706). Particular methods of linking an AE compound to a nucleic acid are known (*e.g.,* US Pat. No. 5,585,481 and US Pat. No. 5,639,604, *see* column 10, line 6 to column 11, line 3, and Example 8). Particular AE labeling positions are a probe's central region and near a region of A/T base pairs, at a probe's 3' or 5' terminus, or at or near a mismatch site with a known sequence that is the probe should not detect compared to the desired target sequence. Other detectably labeled probes include TaqMan™ probes, molecular torches, and molecular beacons. TaqMan™ probes include a donor and acceptor label wherein fluorescence is detected upon enzymatically degrading the probe during amplification in order to release the fluorophore from the presence of the quencher. Molecular torches and beacons exist in open and closed configurations wherein the closed configuration quenches the fluorophore and the open position separates the fluorophore from the quencher to allow fluorescence. Hybridization to a target nucleic acid opens the otherwise closed probes.

By "stably," "stable" or "stable for detection" is meant that the temperature of a reaction mixture is at least 2°C below the melting temperature of a nucleic acid duplex. The temperature of the reaction mixture is more preferably at least 5°C below the melting temperature of the nucleic acid duplex, and even more preferably at least 10°C below the melting temperature of the reaction mixture.

By "substantially homologous," "substantially corresponding" or "substantially corresponds" is meant that the subject oligonucleotide has a base sequence containing an at least 10 contiguous base region that is at least about 80% homologous, preferably at least about 90% homologous, and most preferably 100% homologous to an at least 10 contiguous base region present in a reference base sequence (excluding RNA and DNA equivalents). (Those skilled in the art will readily appreciate modifications that could be made to the hybridization assay conditions at various percentages of homology to permit hybridization of the oligonucleotide to the target sequence while preventing levels of non-specific hybridization sufficient to interfere with detection of the target nucleic acid.) The degree of similarity is determined by comparing the order of nucleobases making up the two sequences and does not take into consideration other structural differences which may exist between the two sequences, provided the structural differences do not prevent hydrogen bonding with complementary bases. The degree of homology between two sequences can also be expressed in terms of the number of base differences between each set of at least 10 contiguous bases being compared, which may be 0, 1 or 2 base differences.

By "substantially complementary" is meant that the subject oligonucleotide has a base sequence containing an at least 10 contiguous base region that is at least 80% complementary, preferably at least 90% complementary, and most preferably 100% complementary to an at least 10 contiguous base region present in a target nucleic acid sequence (excluding RNA and DNA equivalents). Those skilled in the art will readily appreciate modifications that could be made to the hybridization assay conditions at various percentages of complementarity to permit hybridization of the oligonucleotide to the target sequence while preventing levels of non-specific hybridization sufficient to interfere with detection of the target nucleic acid. The degree of complementarity is determined by comparing the order of nucleobases making up the two sequences and does not take into consideration other structural differences which may exist between the two sequences, provided the structural differences do not prevent hydrogen bonding with complementary bases. The degree of complementarity between two sequences can also be expressed in terms of the number of base mismatches present in each set of at least 10 contiguous bases being compared, which may be 0, 1 or 2 base mismatches.

It will be appreciated that there is an implied "about" prior to the temperatures, concentrations, and times discussed in the present disclosure, such that slight and insubstantial deviations are within the scope of the present teachings. In general, the term "about" indicates insubstantial variation in a quantity of a component of a composition not having any significant effect on the activity or stability of the composition. All ranges are to be interpreted as encompassing the endpoints in the absence of express exclusions such as "not including the endpoints"; thus, for example, "within 10-15" includes the values 10 and 15.

By "RNA and DNA equivalents" is meant RNA and DNA molecules having essentially the same complementary base pair hybridization properties. RNA and DNA equivalents can have different sugar moieties (*i.e.,* ribose versus deoxyribose), and further may differ by the presence of uracil in RNA and thymine in DNA. The differences between RNA and DNA equivalents do not contribute to differences in homology because the equivalents have the same degree of complementarity to a particular sequence.

By "RNA and DNA equivalent bases" is meant nucleotide bases having the same complementary base pair hybridization properties in RNA and DNA. Here the base uracil can be substituted in place of the base thymine, or vice versa, and so uracil and thymine are RNA and DNA equivalent bases. A polynucleotide base sequence 5'-AGCT-3' that allows for substitution of RNA and DNA equivalent bases would also describe the sequence 5'-AGCU-3'. The differences between RNA and DNA equivalent bases do not contribute to differences in homology because the equivalents have the same degree of complementarity to a particular sequence.

The term, "complement" refers to a nucleic acid molecule that comprises a contiguous nucleic acid sequence that is complementary to a contiguous nucleic acid sequence of another nucleic acid molecule (for standard nucleotides A:T, A:U, C:G). For example, 5'-AACTGUC-3' is the complement of 5'-GACAGTT-3'.

A "target nucleic acid" (sometimes simply "target") as used herein is a nucleic acid comprising a sequence to be amplified and/or detected. Target nucleic acids may be DNA or RNA, and may be either single-stranded or double-stranded. The target nucleic acid may include other sequences besides the target sequence, which may not be amplified. Examples of target nucleic acids include 16S rRNA and 23S rRNA. The claims may restrict a target sequence to the particular sense of the recited sequence with a proviso excluding complementary sequences.

The term "target sequence" as used herein refers to the particular nucleotide sequence of the target nucleic acid that is to be amplified and/or detected. The "target sequence" includes the complexing sequences to which oligonucleotides (e.g., priming oligonucleotides and/or promoter oligonucleotides) complex during an amplification processes (e.g., PCR, TMA). Where the target nucleic acid is originally single-stranded, the term "target sequence" will also refer to the sequence complementary to the "target sequence" as present in the target nucleic acid. Where the target nucleic acid is originally double-stranded, the term "target sequence" refers to both the sense (+) and antisense (-) strands.

"Target-hybridizing sequence of bases" or "target-hybridizing sequence" or "target-specific sequence" is used herein to refer to the portion of an oligomer that is configured to hybridize with a target nucleic acid sequence. Preferably, the target-hybridizing sequences are configured to specifically hybridize with a target nucleic acid sequence. Target-hybridizing sequences may be 100% complementary to the portion of the target sequence to which they are configured to hybridize, but not necessarily. Target-hybridizing sequences may also include inserted, deleted and/or substituted nucleotide residues relative to a target sequence. Less than 100% complementarity of a target-hybridizing sequence to a target sequence may arise, for example, when the target nucleic acid is a plurality strains within a species, such as would be the case for an oligomer configured to hybridize to sequence variants. It is understood that other reasons exist for configuring a target-hybridizing sequence to have less than 100% complementarity to a target nucleic acid.

By "hybridization" or "hybridize" is meant the ability of two completely or partially complementary nucleic acid strands to come together under specified hybridization assay conditions in a parallel or antiparallel orientation to form a stable structure having a double-stranded region. The two constituent strands of this double-stranded structure, sometimes called a "hybrid," are held together by hydrogen bonds. Although these hydrogen bonds most commonly form between nucleotides containing the bases adenine and thymine or uracil (A and T or U) or cytosine and guanine (C and G) on single nucleic acid strands, base pairing can also form between bases which are not members of these "canonical" pairs. Non-canonical base pairing is well-known in the art. *See, e.g.,* R. L. P. Adams et al., The Biochemistry of the Nucleic Acids (11th ed. 1992).

By "preferentially hybridize" is meant that under stringent hybridization assay conditions, hybridization assay probes can hybridize to their target nucleic acids to form stable probe:target hybrids indicating the presence of at least one organism of interest ("detectable hybrids"), and there is not formed a sufficient number of detectable stable probe:non-target hybrids to indicate the presence of non-targeted organisms ("non-detectable hybrids"), especially phylogenetically closely related organisms. Thus, the probe hybridizes to target nucleic acid to a sufficiently greater extent than to non-target nucleic acid to enable one having ordinary skill in the art to accurately detect the presence (or absence) of nucleic acid derived from *T. pallidum,* and distinguish its presence from that of a phylogenetically closely related organism in a test sample. In general, reducing the degree of complementarity between an oligonucleotide sequence and its target sequence will decrease the degree or rate of hybridization of the oligonucleotide to its target region. However, the inclusion of one or more non-complementary bases may facilitate the ability of an oligonucleotide to discriminate against non-target organisms.

Preferential hybridization can be measured using any of a variety of techniques known in the art, including, but not limited to those based on light emission, mass changes, and changes in conductivity or turbidity. A number of detection means are described herein, and one in particular is used in the Examples provided below. Preferably, there is at least a 10-fold difference between target and non-target hybridization signals in a test sample, more preferably at least a 100-fold difference, and most preferably at least a 500-fold difference. Preferably, non-target hybridization signals in a test sample are no more than the background signal level.

By "stringent hybridization assay conditions," "hybridization assay conditions," "stringent hybridization conditions," or "stringent conditions" is meant conditions permitting a hybridization assay probe to preferentially hybridize to a target nucleic acid (preferably rRNA or rDNA derived from *T. pallidum* over nucleic acid derived from a closely related non-target microorganism. Stringent hybridization assay conditions may vary depending upon factors including the GC content and length of the probe, the degree of similarity between the probe sequence and sequences of non-target sequences which may be present in the test sample, and the target sequence. Hybridization conditions include the temperature and the composition of the hybridization reagents or solutions. While the Examples section *infra* provides preferred hybridization assay conditions for detecting target nucleic acids derived from *T. pallidum* using the probes of the present disclosure, other stringent conditions could be easily ascertained by someone having ordinary skill in the art.

By "assay conditions" is meant conditions permitting stable hybridization of an oligonucleotide to a target nucleic acid. Assay conditions do not require preferential hybridization of the oligonucleotide to the target nucleic acid.

A "homogeneous detectable label" refers to a label that can be detected in a homogeneous fashion by determining whether the label is on a probe hybridized to a target sequence. That is, homogeneous detectable labels can be detected without physically removing hybridized from unhybridized forms of the label or labeled probe. Homogeneous detectable labels are preferred when using labeled probes for detecting amplified nucleic acids. Examples of homogeneous labels have been described in detail by Arnold et al., U.S. Patent No. 5,283,174; Woodhead et al., U.S. Patent No. 5,656,207; and Nelson et al., U.S. Patent No. 5,658,737. Preferred labels for use in homogenous assays include chemiluminescent compounds (*e.g.,* see Woodhead et al., U.S. Patent No. 5,656,207; Nelson et al., U.S. Patent No. 5,658,737; and Arnold, Jr., et al., U.S. Patent No. 5,639,604). Preferred chemiluminescent labels are acridinium ester ("AE") compounds, such as standard AE or derivatives thereof (*e.g.*, naphthyl-AE, ortho-AE, 1- or 3-methyl-AE, 2,7-dimethyl-AE, 4,5-dimethyl-AE, ortho-dibromo-AE, ortho-dimethyl-AE, meta-dimethyl-AE, ortho-methoxy-AE, ortho-methoxy(cinnamyl)-AE, ortho-methyl-AE, ortho-fluoro-AE, 1- or 3-methyl-ortho-fluoro-AE, 1- or 3-methyl-meta-difluoro-AE, and 2-methyl-AE).

A "homogeneous assay" refers to a detection procedure that does not require physical separation of hybridized probe from non-hybridized probe prior to determining the extent of specific probe hybridization. Exemplary homogeneous assays, such as those described herein, can employ molecular torches, molecular beacons, or other self-reporting probes which emit fluorescent signals when hybridized to an appropriate target, chemiluminescent acridinium ester labels which can be selectively destroyed by chemical means unless present in a hybrid duplex, and other homogeneously detectable labels that will be familiar to those having an ordinary level of skill in the art.

By "nucleic acid duplex," "duplex," "nucleic acid hybrid" or "hybrid" is meant a stable nucleic acid structure comprising a double-stranded, hydrogen-bonded region. Such hybrids include RNA:RNA, RNA:DNA and DNA:DNA duplex molecules and analogs thereof. The structure is sufficiently stable to be detectable by any known means.

An "amplification oligonucleotide" or "amplification oligomer" is an oligonucleotide that hybridizes to a target nucleic acid, or its complement, and participates in a nucleic acid amplification reaction (*e.g.*, serving as a primer or promoter-primer). Particular amplification oligomers contain at least about 10 contiguous bases, and optionally at least 17, 18, 19, 20, 21, or 22 contiguous bases that are complementary to a region of the target nucleic acid sequence or its complementary strand. The contiguous bases may be at least about 80%, at least about 90%, or completely complementary to the target sequence to which the amplification oligomer binds. One skilled in the art will understand that the recited ranges include all whole and rational numbers within the range (*e.g.,* 92% or 98.377%). Particular amplification oligomers are about 17 to about 50 bases long, or more preferably about 17 to about 22 bases long and optionally may include modified nucleotides, or an extraneous base sequence not found in *T. pallidum* attached at the 5' terminus.

A "primer" is an amplification oligomer that hybridizes to a template nucleic acid and has a 3' end that is extended by a polymerase enzyme. A primer may be optionally modified (*e.g.*, by including a 5' region that is non-complementary to the target sequence). Such modification can include functional additions, such as tags, promoters, or other non-target-specific sequences used or useful for manipulating or amplifying the primer or target oligonucleotide.

Within the context of Transcription Mediated Amplification, a primer modified with a 5' promoter sequence is referred to herein as a "promoter-primer." A person of ordinary skill in the art of molecular biology or biochemistry will understand that an oligomer that can function as a primer can be modified to include a 5' promoter sequence and then function as a promoter-primer, and, similarly, any promoter-primer can serve as a primer with or without its 5' promoter sequence. A promoter-primer modified to incorporate a 3' blocked end is referred to herein as a "promoter provider," which is capable of hybridizing to a target nucleic acid and providing an upstream promoter sequence that serves to initiate transcription, but does not provide a primer for oligo extension.

"Nucleic acid amplification" or "target amplification" or simply "amplification" refers to any *in vitro* procedure that produces multiple copies of a target nucleic acid sequence, or its complementary sequence, or fragments thereof (*i.e.,* an amplified sequence containing less than the complete target nucleic acid). Examples of nucleic acid amplification procedures include transcription associated methods, such as Transcription Mediated Amplification (TMA), nucleic acid sequence-based amplification (NASBA) and others (*e.g.,* U.S. Patent Nos. 5,399,491, 5,554,516, 5,437,990, 5,130,238, 4,868,105, and 5,124,246), replicase-mediated amplification (*e.g.*, U.S. Patent No. 4,786,600), the polymerase chain reaction (PCR) (*e.g.,* U.S. Patent Nos. 4,683,195, 4,683,202, and 4,800,159), ligase chain reaction (LCR) (*e.g.,* EP Patent No. 0320308), helicase-dependent amplification (*e.g.,* U.S. Patent No. 7,282,328), and strand-displacement amplification (SDA) (*e.g.,* U.S. Patent No. 5,422,252). Amplification may be linear or exponential. PCR amplification uses DNA polymerase, primers, and thermal cycling steps to synthesize multiple copies of the two complementary strands of DNA or cDNA. LCR amplification uses at least four separate oligonucleotides to amplify a target and its complementary strand by using multiple cycles of hybridization, ligation, and denaturation. Helicase-dependent amplification uses a helicase to separate the two strands of a DNA duplex generating single-stranded templates, followed by hybridization of sequence-specific primers hybridize to the templates and extension by DNA polymerase to amplify the target sequence. SDA uses a primer that contains a recognition site for a restriction endonuclease that will nick one strand of a hemimodified DNA duplex that includes the target sequence, followed by amplification in a series of primer extension and strand displacement steps. Replicase-mediated amplification uses self-replicating RNA molecules, and a replicase such as Qβ-replicase. Particular embodiments use PCR or TMA, but it will be apparent to persons of ordinary skill in the art that oligomers disclosed herein may be readily used as primers in other amplification methods.

Transcription-associated amplification uses a DNA polymerase, an RNA polymerase, deoxyribonucleoside triphosphates, ribonucleoside triphosphates, a promoter-containing oligonucleotide, and optionally may include other oligonucleotides, to ultimately produce multiple RNA transcripts from a nucleic acid template (described in detail in, *e.g.,* U.S. Patent Nos. 5,399,491 and 5,554,516 to Kacian et al.*;* U.S. Patent No. 5,437,990 to Burg et al.*;* PCT Publication Nos. WO 88/01302 and WO 88/10315 (Gingeras et al.); U.S. Patent No. 5,130,238 to Malek et al.*;* U.S. Patent Nos. 4,868,105 and 5,124,246 to Urdea et al.*;* PCT Publication No. WO 94/03472 (McDonough et al.); and PCT Publication No. WO 95/03430 (Ryder et al.)). Methods that use TMA are described in detail previously (*e.g.*, US Pat. Nos. 5,399,491 and 5,554,516).

By "amplification conditions" is meant conditions permitting nucleic acid amplification. While the Examples section *infra* provides preferred amplification conditions for amplifying target nucleic acid sequences derived from *T. pallidum* using primers of the present disclosure in a Transcription Mediated Amplification method, other acceptable amplification conditions could be easily determined by one having ordinary skill in the art, depending on the particular method of amplification desired.

By "opposite sense" or "opposite strand" is meant a nucleic acid molecule perfectly complementary to a reference, or sense, nucleic acid strand.

By "sense," "same-sense" or "positive sense" is meant a nucleic acid molecule perfectly homologous to a reference nucleic acid molecule.

By "amplicon" is meant a nucleic acid molecule generated in a nucleic acid amplification reaction and which is derived from a target nucleic acid. An amplicon contains a target nucleic acid sequence that may be of the same or opposite sense as the target nucleic acid. By "derived" is meant that the referred to nucleic acid is obtained directly from a target organism or indirectly as the product of a nucleic acid amplification, which product may be, for instance, an antisense RNA molecule that does not exist in the target organism.

By "capture probe" is meant an oligonucleotide or a set of at least two oligonucleotides linked together which are capable of hybridizing to a target nucleic acid and to an immobilized probe, thereby providing means for immobilizing and isolating the target nucleic acid in a test sample. That portion of the capture probe which hybridizes to the target nucleic acid is referred to as the "target binding region," and that portion of the capture probe which hybridizes to the immobilized probe is referred to as the "immobilized probe binding region." While the preferred capture probe hybridizes to both the target nucleic acid and the immobilized probe under assay conditions, the target binding region and the immobilized probe binding region may be designed to hybridize to their respective target sequences under different hybridization conditions. In this way, the capture probe may be designed so that it first hybridizes to the target nucleic acid under more favorable solution-phase kinetics before adjusting the conditions to permit hybridization of the immobilized probe binding region to the immobilized probe. When the target binding and immobilized probe binding regions are provided on the same capture probe, they may be directly adjoining each other on the same oligonucleotide, they may be separated from each other by one or more optionally modified nucleotides, or they may be joined to each other by means of a non-nucleotide linker.

By "target-hybridizing sequence" or "target binding region" is meant that portion of an oligonucleotide which stably binds to a target sequence present in a target nucleic acid, a DNA or RNA equivalent of the target sequence or a complement of the target sequence under assay conditions. The assay conditions may be stringent hybridization conditions or amplification conditions.

By "immobilized probe binding region" is meant that portion of an oligonucleotide which hybridizes to an immobilized probe under assay conditions.

By "homopolymer tail" in the claims is meant a contiguous base sequence of at least 10 identical bases (*e.g.,* 10 contiguous adenines or thymines).

By "immobilized probe" is meant an oligonucleotide for joining a capture probe to an immobilized support. The immobilized probe is joined either directly or indirectly to the solid support by a linkage or interaction which remains stable under the conditions employed to hybridize the capture probe to the target nucleic acid and to the immobilized probe, whether those conditions are the same or different. The immobilized probe facilitates separation of the bound target nucleic acid from unbound materials in a sample.

By "isolate" or "isolating" is meant that at least a portion of the target nucleic acid present in a test sample is concentrated within a reaction receptacle or on a reaction device or solid carrier (*e.g.,* test tube, cuvette, microtiter plate well, nitrocellulose filter, slide or pipette tip) in a fixed or releasable manner so that the target nucleic acid can be purified without significant loss of the target nucleic acid from the receptacle, device or carrier.

By "separate," "separation," "separating" or "purify," "purified" or "purifying" is meant that one or more components of a sample contained in or on a receptacle, device or carrier are physically removed from one or more other sample components present in or on the receptacle, device or carrier. Sample components which may be removed during a separating or purifying step include proteins, carbohydrates, lipids, inhibitors, non-target nucleic acids and unbound probe. Preferably retained in a sample during a separating or purifying step are target nucleic acids bound to immobilized capture probes.

By "species-specific" is meant that the referred to hybridization assay probe is capable of preferentially detecting (*e.g.,* under stringent hybridization assay conditions) a target nucleic acid sequence present in nucleic acid derived from organisms belonging to the species *T. pallidum.*

By "consisting essentially of" is meant that additional component(s), composition(s) or method step(s) that do not materially change the basic and novel characteristics of the present invention may be included in the compositions or kits or methods of the present invention. Any component(s), composition(s), or method step(s) that have a material effect on the basic and novel characteristics of the present invention would fall outside of this term. For example, additions or deletions to an oligonucleotide can be non-material variations which do not prevent the oligonucleotide from having its claimed property (*i.e.,* preferentially hybridizing under stringent hybridization assay conditions to the target nucleic acid over non-target nucleic acids). The oligonucleotide may include other nucleic acid molecules which do not participate in hybridization of the probe to the target nucleic acid and which do not affect such hybridization.

Hybridization Conditions and Probe Design

Hybridization reaction conditions, most importantly the temperature of hybridization and the concentration of salt in the hybridization solution, can be selected to allow the hybridization assay probes of the present disclosure to preferentially hybridize to nucleic acids having a target nucleic sequence derived from *T. pallidum.* At decreased salt concentrations and/or increased temperatures (conditions of increased stringency) the extent of nucleic acid hybridization decreases as hydrogen bonding between paired nucleotide bases in the double-stranded hybrid molecule is disrupted. This process is known as "melting."

Generally speaking, the most stable hybrids are those having the largest number of contiguous, perfectly matched (*i.e.,* hydrogen-bonded) nucleotide base pairs. Such hybrids would usually be expected to be the last to melt as the stringency of the hybridization conditions increases. However, a double-stranded nucleic acid region containing one or more mismatched, "non-canonical," or imperfect base pairs (resulting in weaker or non-existent base pairing at that position in the nucleotide sequence of a nucleic acid) may still be sufficiently stable under conditions of relatively high stringency to allow the nucleic acid hybrid to be formed and detected in a hybridization assay without cross-reacting with other, non-selected nucleic acids which may be present in a test sample.

Hence, depending on the degree of similarity between the nucleotide sequences of the target nucleic acid and those of non-target nucleic acids belonging to phylogenetically distinct, but closely related organisms on the one hand, and the degree of complementarity between the nucleotide sequences of a particular probe and those of the target and non-target nucleic acids on the other, one or more mismatches will not necessarily defeat the ability of an oligonucleotide contained in the probe or primer to hybridize to the target nucleic acid and not to non-target nucleic acids.

The hybridization assay probes of the present disclosure were chosen, selected, and/or designed to maximize the difference between the melting temperatures ($T_m$) of the probe:target hybrid ($T_m$ is defined as the temperature at which half of the potentially double-stranded molecules in a given reaction mixture are in a single-stranded, denatured state) and the $T_m$ of a mismatched hybrid formed between the probe and rRNA or rDNA of the phylogenetically most closely-related organisms expected to be present in the test sample, but not sought to be detected. While the unlabeled amplification primers and capture probes need not have such an extremely high degree of specificity as the hybridization assay probe to be useful in the present disclosure, they are designed in a similar manner to preferentially hybridize to one or more target nucleic acids over other nucleic acids under specified amplification or hybridization assay conditions. The sequences used for this comparison were determined in the laboratory or obtained from published sources.

Within the rRNA molecule there is a close relationship between secondary structure (caused in part by intra-molecular hydrogen bonding) and function. This fact imposes restrictions on evolutionary changes in the primary nucleotide sequence causing the secondary structure to be maintained. For example, if a base is changed in one "strand" of a double helix (due to intra-molecular hydrogen bonding, both "strands" are part of the same rRNA molecule), a compensating substitution usually occurs in the primary sequence of the other "strand" in order to preserve complementarity (this is referred to as co-variance), and thus the necessary secondary structure. This allows two very different rRNA sequences to be aligned based both on the conserved primary sequence and also on the conserved secondary structure elements. Potential target sequences for the hybridization assay probes described herein were identified by noting variations in the homology of the aligned sequences.

Merely identifying putatively unique potential target nucleotide sequences does not guarantee that a functionally species-specific hybridization assay probe may be made to hybridize to *T. pallidum* rRNA or rDNA including that sequence. Various other factors will determine the suitability of a nucleic acid locus as a target site for species-specific probes. Because the extent and specificity of hybridization reactions, such as those described herein, are affected by a number of factors, manipulation of one or more of those factors will determine the exact sensitivity and specificity of a particular oligonucleotide, whether perfectly complementary to its target or not. The importance and effect of various assay conditions are known to those skilled in the art and are disclosed by the following: Kohne, "Method for Detection, Identification and Quantitation of Non-Viral Organisms," U.S Patent No. 4,851,330; Hogan et al., "Nucleic Acid Probes to Mycobacterium gordonae," U.S. Patent No. 5,216,143; and Hogan, "Nucleic Acid Probes for Detection and/or Quantitation of Non-Viral Organisms," U.S. Patent No. 5,840,488.

The desired temperature of hybridization and the hybridization solution composition (such as salt concentration, detergents and other solutes) can also affect the stability of double-stranded hybrids. Conditions such as ionic strength and the temperature at which a probe will be allowed to hybridize to a target must be taken into account in constructing a species-specific probe. The thermal stability of hybrid nucleic acids generally increases with the ionic strength of the reaction mixture. On the other hand, chemical reagents which disrupt hydrogen bonds, such as formamide, urea, dimethyl sulfoxide and alcohols, can greatly reduce the thermal stability of the hybrids.

To maximize the specificity of a probe for its target, probes of the present disclosure were designed to hybridize to their targets under conditions of high stringency. Under such conditions only single nucleic acid strands (or regions) having a high degree of complementarity will hybridize to each other. Single nucleic acid strands without such a high degree of complementarity will not form hybrids. Accordingly, the stringency of the assay conditions determines the amount of complementarity which should exist between two nucleic acid strands in order to form a hybrid. Stringency is chosen to maximize the difference in stability between the hybrid formed between the probe and the target nucleic acid and potential hybrids between the probe and any non-target nucleic acids present in a test sample.

Proper specificity may be achieved by minimizing the length of the hybridization assay probe having perfect complementarity to sequences of non-target organisms, by avoiding G and C rich regions of complementarity to non-target nucleic acids, and by constructing the probe to contain as many destabilizing mismatches to non-target sequences as possible. Whether a probe is appropriate for detecting only a specific type of organism depends largely on the thermal stability difference between probe:target hybrids versus probe:non-target hybrids. In designing probes, the differences in these $T_m$ values should be as large as possible (preferably 2°C-5°C or more). Manipulation of the $T_m$ can be accomplished by changes to probe length and probe composition, such as GC content versus AT content or the inclusion of nucleotide analogs (*e.g.*, ribonucleotides having a 2'-O-methyl substitution to the ribofuranosyl moiety).

In general, the optimal hybridization temperature for oligonucleotide probes is approximately 5°C below the melting temperature for a given duplex. Incubation at temperatures below the optimum temperature may allow mismatched base sequences to hybridize and can therefore decrease specificity. The longer the probe, the more hydrogen bonding between base pairs and, in general, the higher the $T_m$. Increasing the percentage of G and C also increases the $T_m$ because G-C base pairs exhibit additional hydrogen bonding and therefore greater thermal stability than A-T base pairs. Such considerations are known in the art. *See, e.g.,* J. SAMBROOK ET AL., MOLECULAR CLONING: A LABORATORY MANUAL CH. 11 (2d ed. 1989).

A preferred method for determining $T_m$ measures hybridization using the well known hybridization protection assay (HPA) disclosed by Arnold et al., "Homogenous Protection Assay," U.S. Patent No. 5,283,174. The $T_m$ can be measured using HPA in the following manner. Probe molecules are labeled with an acridinium ester and permitted to form probe:target hybrids in a lithium succinate buffer (0.1 M lithium succinate buffer, pH 4.7, 20 mM EDTA, 15 mM aldrithiol-2, 1.2 M LiCl, 3% (v/v) absolute ethanol, 2% (w/v) lithium lauryl sulfate) using an excess amount of target. Aliquots of the solution containing the probe:target hybrids are then diluted in the lithium succinate buffered solution and incubated for five minutes at various temperatures starting below that of the anticipated $T_m$ (typically 55°C) and increasing in 2-5°C increments. This solution is then diluted with a mild alkaline borate buffer (600 mM boric acid, 240 mM NaOH, 1% (v/v) TRITON® X-100, pH 8.5) and incubated at an equal or lower temperature (*e.g.,* 50°C) for ten minutes.

Under these conditions the acridinium ester attached to the single-stranded probe is hydrolyzed, while the acridinium ester attached to hybridized probe is relatively protected from hydrolysis. Thus, the amount of acridinium ester remaining after hydrolysis treatment is proportional to the number of hybrid molecules. The remaining acridinium ester can be measured by monitoring the chemiluminescence produced from the remaining acridinium ester by adding hydrogen peroxide and alkali to the solution. Chemiluminescence can be measured in a luminometer, such as a LEADER® 450i luminometer (Gen-Probe Incorporated, San Diego, CA). The resulting data can be plotted as percent of maximum signal (usually from the lowest temperature) versus temperature. The $T_m$ is defined as the temperature at which 50% of the maximum signal remains. In addition to the method above, $T_m$ may be determined by isotopic methods known to those skilled in the art (*see, e.g.,* U.S. Patent No. 5,840,488).

It should be noted that the $T_m$ for a given hybrid varies depending on the nature of the hybridization solution used. Factors such as the salt concentration, detergents, and other solutes can affect hybrid stability during thermal denaturation (*see, e.g.,* SAMBROOK ET AL., *supra, ch.* 11). Conditions such as ionic strength and the temperature at which a probe will be allowed to hybridize to target should be taken into account in probe construction. Generally speaking, the thermal stability of a hybrid nucleic acid increases with the ionic strength of the reaction mixture. On the other hand, chemical reagents that disrupt hydrogen bonds, such as formamide, urea, dimethyl sulfoxide and alcohols, can greatly reduce hybrid thermal stability.

To ensure specificity of a hybridization assay probe for its target, it is preferable to design probes which hybridize only to target nucleic acid under conditions of high stringency. Only highly complementary sequences will form hybrids under conditions of high stringency. Accordingly, the stringency of the assay conditions determines the amount of complementarity needed between two sequences in order for a stable hybrid to form. Stringency should be chosen to maximize the difference in stability between the probe:target hybrid and potential probe: non-target hybrids.

Examples of specific stringent hybridization conditions are provided herein. Of course, alternative stringent hybridization conditions could be determined by those of ordinary skill in the art based on the present disclosure. (*See, e.g.,* SAMBROOK ET AL., *supra, ch.* 11.)

The length of the target nucleic acid sequence region and, accordingly, the length of the probe sequence can also be important. In some cases, there may be several sequences from a particular region, varying in location and length, which may be used to design probes with the desired hybridization characteristics. In other cases, one probe may be significantly better with regard to specificity than another which differs from it merely by a single base. While it is possible for nucleic acids that are not perfectly complementary to hybridize, the longest stretch of perfectly complementary bases, as well as the base compositions, will generally determine hybrid stability.

Regions of rRNA known to form strong internal structures inhibitory to hybridization are less preferred target regions. Likewise, probes with extensive self-complementarity are generally to be avoided. However, some degree of self-complementarity in a probe may be desirable, as in hairpin probes like the molecular torches and molecular beacons discussed herein. If a strand is wholly or partially involved in an intra-molecular or inter-molecular hybrid, it will be less able to participate in the formation of a new inter-molecular probe:target hybrid without a change in the reaction conditions. Ribosomal RNA molecules are known to form very stable intra-molecular helices and secondary structures by hydrogen bonding. By designing a probe to a region of the target nucleic acid which remains substantially single-stranded under hybridization conditions, the rate and extent of hybridization between probe and target may be increased.

A genomic ribosomal nucleic acid (rDNA) target occurs naturally in a double-stranded form, as does a product of the polymerase chain reaction (PCR). These double-stranded targets are naturally inhibitory to hybridization with a probe and require denaturation prior to hybridization. Appropriate denaturation and hybridization conditions are known in the art (*see, e.g.,* Southern, E.M., J. Mol. Biol., 98:503 (1975)).

A number of formulae are available which will provide an estimate of the melting temperature for perfectly matched oligonucleotides to their target nucleic acids. One such formula is the following:

$$T_m = 81.5 + 16.6(\log_{10}[\text{Na}^+]) + 0.41(\text{fraction G+C}) - (600/N)$$

(where $N$ = the length of the oligonucleotide in number of nucleotides) provides a good estimate of the $T_m$ for oligonucleotides between 14 and 60 to 70 nucleotides in length. From such calculations, subsequent empirical verification or "fine tuning" of the $T_m$ may be made using screening techniques well known in the art. For further information on hybridization and oligonucleotide probes, reference may be made to SAMBROOK ET AL., *supra,* ch. 11. This reference, among others well known in the art, also provides estimates of the effect of mismatches on the $T_m$ of a hybrid. Thus, from the known nucleotide sequence of a given region of the ribosomal RNA (or rDNA) of two or more organisms, oligonucleotides may be designed which will distinguish these organisms from one another.

Preparation of Oligonucleotides

The hybridization assay probes, amplification primers and capture probes of the present disclosure can be readily prepared by methods known in the art. Preferably, the oligonucleotides are synthesized using solid phase methods. Standard phosphoramidite solid-phase chemistry for joining nucleotides by phosphodiester linkages is disclosed by Caruthers et al., in "Chemical Synthesis of Deoxynucleotides by the Phosphoramidite Method," Methods Enzymol., 154:287 (1987). Automated solid-phase chemical synthesis using cyanoethyl phosphoramidite precursors has been described by Barone. *See* Barone et al., "In Situ Activation of bis-dialkylaminephosphines -- a New Method for Synthesizing Deoxyoligonucleotides on Polymer Supports," Nucleic Acids Res., 12(10):4051 (1984). Batt discloses a procedure for synthesizing oligonucleotides containing phosphorothioate linkages in U.S. Patent No. 5,449,769, entitled "Method and Reagent for Sulfurization of Organophosphorous Compounds." In addition, Riley *et al.* disclose the synthesis of oligonucleotides having different linkages including methylphosphonate linkages in U.S. Patent No. 5,811,538, entitled "Process for the Purification of Oligomers." Moreover, methods for the organic synthesis of oligonucleotides are known to those of skill in the art and are described in, for example, SAMBROOK ET AL., *supra, ch.* 10.

Following synthesis and purification of a particular oligonucleotide, several different procedures may be utilized to purify and control the quality of the oligonucleotide. Suitable procedures include polyacrylamide gel electrophoresis or high pressure liquid chromatography. Both of these procedures are well known to those skilled in the art.

All of the oligonucleotides of the present disclosure, whether hybridization assay probes, amplification primers or capture probes, may be modified with chemical groups to enhance their performance or to facilitate the characterization of amplification products. For example, backbone-modified oligonucleotides such as those having phosphorothioate, methylphosphonate, 2'-O-alkyl or peptide groups which render the oligonucleotides resistant to the nucleolytic activity of certain polymerases or to nuclease enzymes may allow the use of such enzymes in an amplification or other reaction. Another example of a modification involves using non-nucleotide linkers incorporated between nucleotides in the nucleic acid chain of a probe or primer, and which do not prevent hybridization of a probe or hybridization and elongation of a primer. *See* Arnold et al., "Non-Nucleotide Linking Reagents for Nucleotide Probes," U.S. Patent No. 6,031,091. The oligonucleotides of the present disclosure may also contain mixtures of the desired modified and natural nucleotides.

The 3' end of an amplification oligonucleotide can be modified or blocked to prevent or inhibit initiation of DNA synthesis, as disclosed by Kacian et al. in U.S. Patent No. 5,554,516. The 3' end of the amplification oligonucleotide can be modified in a variety of ways well known in the art. By way of example, appropriate modifications can include the addition of ribonucleotides, 3' deoxynucleotide residues (*e.g.*, cordycepin), 2',3'-dideoxynucleotide residues, modified nucleotides such as phosphorothioates, and non-nucleotide linkages such as those disclosed by Arnold et al. in U.S. Patent No. 6,031,091 or alkane-diol modifications (*see* Wilk et al., "Backbone-Modified Oligonucleotides Containing a Butanediol-1,3 Moiety as a 'Vicarious Segment' for the Deoxyribosyl Moiety -- Synthesis and Enzyme Studies," Nucleic Acids Res., 18(8):2065 (1990)), or the modification may simply consist of a region 3' to the priming sequence that is non-complementary to the target nucleic acid sequence. Additionally, a mixture of different 3' blocked primers or of 3' blocked and unblocked primers may increase the efficiency of nucleic acid amplification, as disclosed therein.

The 5' end of primers can be modified to be resistant to the 5'-exonuclease activity present in some nucleic acid polymerases. Such modifications can be carried out by adding a non-nucleotide group to the terminal 5' nucleotide of the primer using techniques such as those disclosed by Arnold et al. in U.S. Patent No. 6,031,091. To facilitate strand displacement, the 5' end may also be modified to include non-complementary nucleotides as disclosed by Dattagupta et al, "Isothermal Strand Displacement Nucleic Acid Amplification," U.S. Patent No. 6,087,133.

Once synthesized, a selected oligonucleotide may be labeled by any of several well-known methods (*see, e.g.,* SAMBROOK, *supra, ch.* 10). Useful labels include radioisotopes as well as non-radioactive reporting groups. Isotopic labels include $^{3}$H, $^{35}$S, $^{32}$P, $^{125}$I, $^{57}$Co and $^{14}$C. Isotopic labels can be introduced into the oligonucleotide by techniques known in the art such as nick translation, end labeling, second strand synthesis, the use of reverse transcription, and by chemical methods. When using radiolabeled probes, hybridization can be detected by autoradiography, scintillation counting or gamma counting. The detection method selected will depend upon the particular radioisotope used for labeling.

Non-isotopic materials can also be used for labeling, and may be introduced internally into the nucleic acid sequence or at the end of the nucleic acid sequence. Modified nucleotides may be incorporated enzymatically or chemically. Chemical modifications of the probe may be performed during or after synthesis of the probe, for example, through the use of non-nucleotide linker groups, as disclosed by Arnold et al. in U.S. Patent No. 6,031,091. Non-isotopic labels include fluorescent molecules (individual labels or combinations of interacting labels, such as the fluorescence resonance energy transfer (FRET) pairs disclosed by Tyagi et al. in U.S. Patent No. 5,925,517), chemiluminescent molecules, enzymes, cofactors, enzyme substrates, haptens or other ligands. In some embodiments, the hybridization assay probes of the present disclosure are labeled by means of a non-nucleotide linker with an acridinium ester (AE), such as standard AE. Acridinium ester labeling can be performed as disclosed by Arnold et al., "Acridinium Ester Labelling and Purification of Nucleotide Probes," U.S. Patent No. 5,185,439.

Nucleic Acid Amplification

Preferably, the amplification primers of the present disclosure are oligomers sufficiently long to be used as a substrate for extension by a nucleic acid polymerase. Optimal primer length should take into account several factors, including the temperature of reaction, the structure and base composition of the primer, and how the primer is to be used. For example, for optimal specificity the oligonucleotide primer generally should be at least 12 bases in length, depending on the complexity of the target nucleic acid sequence. If such specificity is not essential, shorter primers may be used. In such a case, it may be desirable to carry out the reaction at lower temperatures in order to form stable hybrid complexes with the template nucleic acid.

Useful guidelines for designing amplification primers with desired characteristics are described above in the section entitled "Preparation of Oligonucleotides." Optimal sites for amplifying and probing contain at least two, and preferably three, conserved regions of *T. pallidum* nucleic acid. These regions are about 15 to 350 bases in length, and preferably between about 15 and 150 bases in length.

The degree of amplification observed with a set of amplification primers (primers and/or promoter-primers) depends on several factors, including the ability of the primers to hybridize to their specific target sequences and their ability to be extended or copied enzymatically. While amplification primers of different lengths and base compositions may be used, amplification primers preferred in this disclosure have target binding regions of 17 to 22 bases, preferably with a predicted $T_m$ to target above 42°C, preferably at least about 50°C.

Parameters affecting probe hybridization, such as melting temperature, complementarity and secondary structure of the target sequence, also affect amplification primer hybridization and therefore performance of the amplification primers. The degree of non-specific extension (primer-dimer or non-target copying) can also affect amplification efficiency. Thus, amplification primers are generally selected to have low self-complementarity or cross-complementarity, particularly at the 3' ends of their sequences. Amplification primers including regions of self-complementarity may be useful, such as the self-reporting "signal primers" disclosed by Nadeau et al., "Detection of Nucleic Acids by Fluorescence Quenching," U.S. Patent No. 5,958,700, and the "hairpin primers" disclosed by Nazarenko et al., "Nucleic Acid Amplification Oligonucleotides with Molecular Energy Transfer Labels and Methods Based Thereon," U.S. Patent No. 5,866,336. Lengthy homopolymer runs and high GC content are avoided to reduce spurious primer extension. Computer programs are available to aid in this aspect of the design, including Oligo Tech® analysis software available from Oligo Therapeutics, Inc.

A nucleic acid polymerase used in conjunction with the amplification primers of the present disclosure refers to a chemical, physical or biological agent which incorporates either ribonucleotides or deoxyribonucleotides, or both, into a nucleic acid polymer, or strand, in a template-dependent manner. Examples of nucleic acid polymerases include DNA-directed DNA polymerases, RNA-directed DNA polymerases, and RNA-directed RNA polymerases. DNA polymerases bring about nucleic acid synthesis in a template-dependent manner and in a 5' to 3' direction. Because of the typical anti-parallel orientation of the two strands in a double-stranded nucleic acid, this direction is from a 3' region on the template to a 5' region on the template. Examples of DNA-directed DNA polymerases include *E. coli* DNA polymerase I, the thermostable DNA polymerase from *Thermus aquaticus* (*Taq*), and the large fragment of DNA polymerase I from *Bacillus stearothermophilus* (*Bst*). *See, e.g.,* Riggs et al., "Purified DNA Polymerase from Bacillus stearothermophilus," U.S. Patent No. 6,066,483. Examples of RNA-directed DNA polymerases include various retroviral reverse transcriptases, such as Moloney murine leukemia virus (MMLV) reverse transcriptase or avian myeloblastosis virus (AMV) reverse transcriptase.

During most nucleic acid amplification reactions, a nucleic acid polymerase adds nucleotide residues to the 3' end of the primer using the target nucleic acid as a template, thus synthesizing a second nucleic acid strand having a nucleotide sequence partially or completely complementary to a region of the target nucleic acid. In many nucleic acid amplification reactions, the two strands comprising the resulting double-stranded structure must be separated by chemical or physical means in order to allow the amplification reaction to proceed. Alternatively, the newly-synthesized template strand may be made available for hybridization with a second primer or promoter-primer by other means, such as through strand displacement or the use of a nucleolytic enzyme which digests part or all of the original target strand. In this way the process may be repeated through a number of cycles, resulting in a large increase in the number of nucleic acid molecules having the target nucleotide sequence.

Either the first or second amplification primer, or both, may be a promoter-primer. In some applications, the amplification primers may only consist of promoter-primers which are complementary to the sense strand, as disclosed by Kacian et al., "Nucleic Acid Sequence Amplification Method, Composition and Kit," U.S. Patent No. 5,554,516. A promoter-primer usually contains an oligonucleotide segment that is not complementary to a nucleotide sequence present in the target nucleic acid molecule or primer extension product(s) (*see, e.g.,* Kacian et al., "Nucleic Acid Sequence Amplification Methods," U.S. Patent No. 5,399,491). These non-complementary sequences may be located 5' to the complementary sequences on the amplification primer and may provide a locus for initiation of RNA synthesis when made double-stranded through the action of a nucleic acid polymerase. The promoter thus provided may allow for the *in vitro* transcription of multiple RNA copies of the target nucleic acid sequence. It will be appreciated that all references to primers herein are inclusive of primers and promoter-primers, unless the context clearly indicates otherwise.

A preferred amplification method is the Transcription Mediated Amplification method disclosed by Kacian et al., "Nucleic Acid Sequence Amplification Methods," U.S. Patent No. 5,480,784. In accord with this method, a promoter-primer having a 3' region complementary to a portion of the target and a 5' promoter region and a primer having the same nucleotide sequence as a portion of the target are contacted with a target RNA molecule. The primer and promoter-primer define the boundaries of the target region to be amplified, including both the sense present on the target molecule and its complement, and thus the length and sequence of the amplicon. In this preferred embodiment, the amplification oligonucleotides and immobilized target RNA are contacted in the presence of effective amounts of Moloney murine leukemia virus-derived reverse transcriptase and T7 RNA polymerase, both ribonucleotide and deoxyribonucleotide triphosphates, and necessary salts and cofactors at 42°C. Under these conditions, nucleic acid amplification occurs, resulting predominantly in the production of RNA amplicons of a sense opposite to that of the target nucleic acid. These amplicons can then be detected in solution by, for example, using one or more detectably labeled hybridization probes. In some embodiments, the hybridization probe is an acridinium ester-labeled hybridization probe of the same sense as the target nucleic acid, and can be detected using the HPA technique, as disclosed by Arnold et al. in U.S. Patent No. 5,283,174.

The 3' terminus of the immobilized probe and the capture probe are preferably "capped" or blocked to prevent or inhibit their use as substrates for nucleic acid polymerase activity. Capping may involve adding 3' deoxyribonucleotides (such as cordycepin), 3', 2'-dideoxynucleotide residues, non-nucleotide linkers, such as those disclosed by Arnold et al. in U.S. Patent No. 6,031,091, alkane-diol modifications, or non-complementary nucleotide residues at the 3' terminus.

Those skilled in the art will recognize that the above-described methodology is amenable, either as described or with obvious modifications, to various other amplification schemes, including, for example, the polymerase chain reaction (PCR), Qβ replicase-mediated amplification, self-sustained sequence replication (3SR), strand displacement amplification (SDA), nucleic acid sequence-based amplification (NASBA), loop-mediated isothermal amplification (LAMP), and the ligase chain reaction (LCR).

Sample Processing

Sample processing prior to amplification or detection of a target sequence may be necessary or useful for discriminating a target sequence from non-target nucleic acid present in a sample. Sample processing procedures may include, for example, direct or indirect immobilization of nucleic acids and/or oligonucleotides from the solution-phase in a heterogeneous assay. With some procedures, such immobilization may require multiple hybridization events. Ranki et al., in "Detection of Microbial Nucleic Acids by a One-Step Sandwich Hybridization Test," U.S. Patent Nos. 4,486,539 and 4,563,419, for example, disclose a one-step nucleic acid "sandwich" hybridization method involving the use of a solid-phase bound nucleic acid having a target complementary sequence and a labeled nucleic acid probe which is complementary to a distinct region of the target nucleic acid. Stabinsky, "Methods and Kits for Performing Nucleic Acid Hybridization Assays," U.S. Patent No. 4,751,177, discloses methods including a "mediator" polynucleotide that reportedly overcomes sensitivity problems associated with the method of Ranki resulting from leakage of immobilized probe from the solid support. Instead of directly immobilizing the target nucleic acid, the mediator polynucleotides of Stabinsky are used to bind and indirectly immobilize target polynucleotide:probe polynucleotide complexes which have formed free in solution.

Any known solid support may be used for sample processing, such as matrices and particles free in solution. The solid support may be, for example, nitrocellulose, nylon, glass, polyacrylate, mixed polymers, polystyrene, silane polypropylene and, preferably, magnetically attractable particles to facilitate recovering sample and/or removing unbound nucleic acids or other sample components. Particularly preferred supports are magnetic spheres that are monodisperse (*i.e.,* uniform in size $\pm$ 5%), thereby providing consistent results, which is particularly advantageous for use in an automated procedure. One such automated procedure is disclosed by Ammann et al., "Automated Process for Isolating and Amplifying a Target Nucleic Acid Sequence," U.S. Patent No. 6,335,166.

An oligonucleotide for immobilizing a target nucleic acid on a solid support may be joined directly or indirectly to the solid support by any linkage or interaction which is stable under assay conditions (*e.g.*, conditions for amplification and/or detection). Referred to herein as an "immobilized probe," this oligonucleotide may bind directly to the target nucleic acid or it may include a base sequence region, such as a homopolymeric tract (*e.g.,* a poly dT) or a simple short repeating sequence (*e.g.,* an AT repeat), which hybridizes to a complementary base sequence region present on a capture probe. Direct joining occurs when the immobilized probe is joined to the solid support in the absence of an intermediate oligonucleotide. For example, direct joining may be via a covalent linkage, chelation or ionic interaction. Indirect joining occurs when the immobilized probe is joined to the solid support by one or more linkers. A "linker" is a means for binding at least two different molecules into a stable complex and contains one or more components of a binding partner set.

Members of a binding partner set are able to recognize and bind to each other. Binding partner sets may be, for example, receptor and ligand, enzyme and substrate, enzyme and cofactor, enzyme and coenzyme, antibody and antigen, sugar and lectin, biotin and streptavidin, ligand and chelating agent, nickel and histidine, substantially complementary oligonucleotides, and complementary homopolymeric nucleic acids or homopolymeric portions of polymeric nucleic acids. Components of a binding partner set are the regions of the members that participate in binding.

A preferred sample processing system having practical advantages in terms of its ease of use and rapidity includes an immobilized probe containing a base sequence which is complementary to a base sequence of a capture probe, referred to herein as an "immobilized probe binding region." The capture probe additionally contains a base sequence, referred to herein as a "target binding region," which may specifically hybridize to a target sequence contained in a target nucleic acid under assay conditions. While specificity of the target binding region of the capture probe for a region of the target nucleic acid is desirable to minimize the number of non-target nucleic acids remaining from the sample after a separation step, it is not a requirement of the capture probes of the present disclosure if the capture probes are being used solely to isolate target nucleic acid. If the capture probe is not being employed to isolate a target nucleic acid for subsequent amplification of a target sequence, the capture probe may further include a detectable label attached within or near the target binding region, such as a substituted or unsubstituted acridinium ester. The labeled capture probe may be used in a homogeneous or semi-homogenous assay to specifically detect hybrid nucleic acids without detecting single-stranded nucleic acids, such as the capture probe.

An advantage of this system is that only a single target-specific hybridization event (capture probe:target) is necessary for target detection, rather than multiple such events (*e.g.*, capture probe:target and probe:target or probe:amplicon) which are required in other sample processing procedures described herein. Also, fewer oligonucleotides in an assay tend to make the assay faster and simpler to optimize, since the overall rate at which a target nucleic acid is captured and detected is limited by the slowest hybridizing oligonucleotide. While the target binding region of a capture probe may be less specific in alternative assay systems, it must still be rare enough to avoid significant saturation of the capture probe with non-target nucleic acids. Thus, the requirement that two separate and specific target sequences be identified in these alternative systems could place constraints on the identification of an appropriate target. By contrast, only one such target sequence is needed when the capture probe simultaneously functions as the detection probe.

Whichever approach is adopted, the assay can include a means for detecting the presence of the target nucleic acid in the test sample. A variety of means for detecting target nucleic acids are well known to those skilled in the art of nucleic acid detection, including means which do not require the presence of a detectable label. Nevertheless, probes including a detectable label are preferred. A labeled probe for detecting the presence of a target nucleic acid would have to include a base sequence which is substantially complementary and specifically hybridizes to a target sequence contained in the target nucleic acid. Once the probe stably binds to the target nucleic acid, and the resulting target:probe hybrid has been directly or indirectly immobilized, non-bound probe can be washed away or inactivated, and the remaining bound probe can be detected and/or measured.

Preferred sample processing systems combine the elements of detection and nucleic acid amplification. These systems first directly or indirectly immobilize a target nucleic acid (*e.g.,* using a capture probe), the captured target nucleic acid is purified by removing cellular debris, non-target nucleic acid and amplification inhibitors from the sample-containing vessel, which is followed by amplification of a target sequence contained in the target nucleic acid. Amplified product is then detected, preferably in solution with a labeled probe. The target nucleic acid may remain in the immobilized state during amplification, or it may be eluted or separated from the solid support prior to amplification using appropriate conditions, such as by first incubating at a temperature above the $T_m$ of the capture probe:target complex and/or the $T_m$ of the capture probe:immobilized probe complex. A preferred embodiment of this system is disclosed by Weisburg et al., "Two-Step Hybridization and Capture of a Polynucleotide," U.S. Patent No. 6,110,678. In this system, the capture probe hybridizes to the target nucleic acid and an immobilized probe hybridizes to the capture probe:target complex under different hybridization conditions. Under a first set of hybridization conditions, hybridization of the capture probe to the target nucleic acid is favored over hybridization of the capture probe to the immobilized probe. Thus, under this first set of conditions, the capture probe is in solution rather than bound to a solid support, thereby maximizing the concentration of the free capture probe and utilizing favorable solution-phase kinetics for hybridization to the target nucleic acid. After the capture probe has had sufficient time to hybridize to the target nucleic acid, a second set of hybridization conditions is imposed permitting in the capture probe:target complex to hybridize to the immobilized probe, thereby isolating the target nucleic acid in the sample solution. The immobilized target nucleic acid may then be purified, and a target sequence present in the target nucleic acid may be amplified and detected. A purification procedure that includes one or more wash steps is generally desirable when working with crude samples (*e.g.*, clinical, environmental, industrial, food, water, etc.) to prevent enzyme inhibition and/or nucleic acid degradation due to substances present in the sample.

## Capture Probes for Isolating Ribosomal Nucleic Acid

Capture probes of the present disclosure are designed to bind to and isolate nucleic acid derived from the 23S ribosomal nucleic acid of *T. pallidum* in the presence of non-target nucleic acid. As such, the capture probes include both a target binding region and an immobilized probe binding region. The target binding region of the capture probes includes a base sequence which hybridizes to a target sequence derived from the 23S ribosomal nucleic acid from *T. pallidum* under assay conditions. While not essential, the target binding region preferably exhibits specificity for the target sequence in the presence of non-target nucleic acid under assay conditions. The immobilized probe binding region has a base sequence which hybridizes to an immobilized probe comprising a polynucleotide, or a chimera containing polynucleotide sequences, which is joined to a solid support present in the test sample, either directly or indirectly. The target binding region and the immobilized probe binding region may be joined to each other directly or by means of, for example, a nucleotide base sequence, an abasic sequence or a non-nucleotide linker.

In a preferred embodiment, capture probes according to the present disclosure include a target binding region comprising a base sequence region which is at least about 85% homologous (preferably at least about 90% homologous, more preferably at least about 95% homologous, and most preferably 100% homologous) to one of the capture probes disclosed herein. The immobilized probe binding region of these preferred capture probes includes a base sequence which hybridizes to an immobilized probe joined directly or indirectly to a solid support provided to the test sample under assay conditions. The immobilized probe binding region preferably comprises a homopolymeric region (*e.g.,* poly (dA)) located at the 3' end of the capture probe which is complementary to a homopolymeric region (*e.g.,* poly (dT)) located at the 5' end of the immobilized probe. Other base sequences may be incorporated into the immobilized probe binding region, including, for example, short repeating sequences.

To prevent undesirable cross-hybridization reactions, the capture probes of the present disclosure preferably exclude nucleotide base sequences, other than the nucleotide base sequence of the target binding region, which can stably bind to nucleic acid derived from any organism which may be present in the test sample under assay conditions. Consistent with this approach, and in order to maximize the immobilization of capture probe:target complexes which are formed, the nucleotide base sequence of the immobilized probe binding region is preferably designed so that it can stably bind to a nucleotide base sequence present in the immobilized probe under assay conditions and not to nucleic acid derived from any organism which may be present in the test sample.

The target binding region and the immobilized probe binding region of the capture probe may be selected so that the capture probe:target complex has a higher $T_m$ than the $T_m$ of the capture probe:immobilized probe complex. In this way, a first set of conditions may be imposed which favors hybridization of the capture probe to the target sequence over the immobilized probe, thereby providing for optimal solution-phase hybridization kinetics for hybridization of the capture probe to the target sequence. Once sufficient time has passed for the capture probe to bind to the target sequence, a second set of less stringent conditions may be imposed which allows for hybridization of the capture probe to the immobilized probe. Sets of conditions useful in these applications can be established by those skilled in the art using no more than routine experimentation.

Capture probes of the present disclosure may also include a label or a pair of interacting labels for direct detection of the target sequence in a test sample. Non-limiting examples of labels, combinations of labels and means for labeling probes are set forth above.

Despite their application in a direct detection assay, the most common use of capture probes is in the isolation and purification of target nucleic acid prior to amplifying a target sequence contained in the target nucleic acid. By isolating and purifying the target nucleic acid prior to amplification, the number of unintended amplification reactions (*i.e.,* amplification of non-target nucleic acid) advantageously can be reduced. To prevent or inhibit the capture probe itself from functioning as a substrate for nucleic acid polymerase activity in the presence of amplification reagents and under amplification conditions, the 3' end of the capture probe can be capped or blocked. Examples of capping agents include 3' deoxyribonucleotides, 3', 2'-dideoxynucleotide residues, non-nucleotide linkers, alkane-diol modifications, and non-complementary nucleotide residues at the 3' terminus.

Certain Examples which follow incorporated a target capture step to isolate and purify target nucleic acid prior to amplification of a target nucleic acid sequence. The capture probes of these examples had 5' target binding regions of SEQ ID NO:34, SEQ ID NO:35, and SEQ ID NO:36, and further included a 3' immobilized probe binding region having a poly dA tail 30 nucleotides in length, as given by SEQ ID NO:37, SEQ ID NO:38, and SEQ ID NO:39. The target binding region of the capture probe was designed to bind to a region of the target nucleic acid distinct from the regions bound by the primer, promoter-primer and hybridization assay probe. The solid support of this target capture assay can be a Sera-Mag™ MG-CM Carboxylate Modified (Seradyn, Inc.; Indianapolis, Indiana; Cat. No. 24152105-050450), 1 micron, super-paramagnetic particle having a covalently bound oligo(dT)$_{14}$ which was able to bind to the poly dA tail of the capture probe under hybridization conditions. Similar magnetic particles are disclosed by Sutor, "Process for Preparing Magnetically Responsive Microparticles," U.S. Patent No. 5,648,124. To draw the particles out of suspension and immobilize them along the inner wall of the sample tubes, the tubes were transferred to a magnetic separation rack disclosed by Acosta et al. in U.S. Patent No. 6,254,826. While the particles were immobilized, fluid was aspirated from the tubes and the tubes were washed with the Wash Buffer described below. The wash step can be repeated before adding the below-described Amplification Reagent and the Enzyme Reagent for amplifying the target sequence. Between wash steps, the particles can be resuspended in the Wash Buffer.

Amplification of *T. pallidum* Ribosomal Nucleic Acid

The presently disclosed amplification primers are directed to regions of the 23S ribosomal nucleic acid derived from *T. pallidum.* The amplification primers may flank, overlap or be contained within at least one of the target nucleic acid sequences of a hybridization assay probe (or its complement) used to detect the presence of *T. pallidum* using a nucleic acid amplification assay. As indicated above, the amplification primers may also include non-complementary bases at their 5' ends, and optionally may comprise a promoter sequence *(e.g.,* a T7 promoter sequence) able to bind an RNA polymerase and direct RNA transcription using the target nucleic acid as a template.

Amplification primers of the present disclosure are capable of amplifying a target nucleic acid sequence present in nucleic acid derived from *T. pallidum.* First strand amplification primers comprise an oligonucleotide having a target-hybridizing sequence of bases that comprises or consists of 18-22 contiguous bases of either SEQ ID NO:3, SEQ ID NO:4, or SEQ ID NO:5, optionally including a 5' promoter sequence *(e.g.,* SEQ ID NO:9). Exemplary first strand primers have the target-hybridizing sequences of SEQ ID NO:8, SEQ ID NO:7, or SEQ ID NO:6. Second strand amplification primers comprise an oligonucleotide having a target-hybridizing sequence of bases that comprises or consists of 17-20 contiguous bases of SEQ ID NO: 13, SEQ ID NO:14, or SEQ ID NO:15. Exemplary second strand primers have the target-hybridizing sequences of SEQ ID NO:18, SEQ ID NO:17, or SEQ ID NO:16.

Amplification primers of the present disclosure may have modifications, such as blocked 3' and/or 5' termini (as discussed above) or sequence additions including, but not limited to, a specific nucleotide sequence recognized by an RNA polymerase *(e.g.,* a promoter sequence for T7, T3 or SP6 RNA polymerase), a sequence which enhances initiation or elongation of RNA transcription by an RNA polymerase, or a sequence which may provide for intra-molecular base pairing and encourage the formation of secondary or tertiary nucleic acid structures.

Amplification primers are used in a nucleic acid amplification procedure, such as the polymerase chain reaction (PCR), Qβ replicase-mediated amplification, self-sustained sequence replication (3SR), Transcription Mediated Amplification (TMA), nucleic acid sequence-based amplification (NASBA), ligase chain reaction (LCR), strand displacement amplification (SDA) and loop-mediated isothermal amplification (LAMP), each of which is well known in the art. *See, e.g.,* Mullis, "Process for Amplifying Nucleic Acid Sequences," U.S. Patent No. 4,683,202; Erlich et al., "Kits for Amplifying and Detecting Nucleic Acid Sequences," U.S. Patent No. 6,197,563; Walker et al., "Strand Displacement Amplification -- an Isothermal, In Vitro DNA Amplification Technique," Nucleic Acids Res., 20(7):1691-1696 (1992); Fahy et al., "Self-sustained Sequence Replication (3SR): An Isothermal Transcription-Based Amplification System Alternative to PCR," PCR Methods and Applications, 1:25-33 (1991); Kacian et al., U.S. Patent No. 5,399,491; Davey et al., "Nucleic Acid Amplification Process," U.S. Patent No. 5,554,517; Birkenmeyer et al., "Amplification of Target Nucleic Acids Using Gap Filling Ligase Chain Reaction," U.S. Patent No. 5,427,930; Marshall et al., "Amplification of RNA Sequences Using the Ligase Chain Reaction," U.S. Patent No. 5,686,272; Walker, "Strand Displacement Amplification," U.S. Patent No. 5,712,124; Notomi et al., "Process for Synthesizing Nucleic Acid," U.S. Patent No. 6,410,278; Dattagupta et al., "Isothermal Strand Displacement Amplification," U.S. Patent No. 6,214,587; and Helen H. Lee et al., Nucleic Acid Amplification Technologies: Application to Disease Diagnosis (1997). Other amplification procedures not specifically indicated but which meet the definition of "nucleic acid amplification" are also contemplated by the inventors.

Amplification primers of the present disclosure are preferably unlabeled but may include one or more reporter groups to facilitate detection of a target nucleic acid in combination with or exclusive of a hybridization assay probe. A wide variety of methods are available to directly detect an amplified target sequence. For example, the nucleotide substrates or the primers can include a detectable label which is incorporated into newly synthesized DNA. The resulting labeled amplification product is then generally separated from the unused labeled nucleotides or primers and the label is detected in the separated product fraction. *See, e.g.,* Wu, "Detection of Amplified Nucleic Acid Using Secondary Capture Oligonucleotides and Test Kit," U.S. Patent No. 5,387,510.

A separation step is not required if the primer is modified by, for example, linking it to two dyes which form a donor/acceptor dye pair *(i.e.,* a pair of interactive labels). The modified primer can be designed so that the fluorescence of one dye pair member remains quenched by the other dye pair member, as long as the primer does not hybridize to target nucleic acid, thereby physically separating the two dyes. Moreover, the primer can be further modified to include a restriction endonuclease recognition site positioned between the two dyes so that when a hybrid is formed between the modified primer and target nucleic acid, the restriction endonuclease recognition site is rendered double-stranded and available for cleavage or nicking by the appropriate restriction endonuclease. Cleavage or nicking of the hybrid then separates the two dyes, resulting in a change in fluorescence due to decreased quenching which can be detected as an indication of the presence of the target organism or organisms in the test sample. Such modified primers are disclosed by Nadeau et al., "Detection of Nucleic Acids by Fluorescence Quenching," U.S. Patent Nos. 5,958,700 and 6,054,279.

Substances which can serve as useful detectable labels are well known in the art and include radioactive isotopes, fluorescent molecules, chemiluminescent molecules, chromophores, as well as ligands such as biotin and haptens which, while not directly detectable, can be readily detected by a reaction with labeled forms of their specific binding partners *(e.g.,* avidin and antibodies, respectively).

Another approach is to detect the amplification product by hybridization with a detectably labeled probe and measuring the resulting hybrids in any conventional manner. For example, the product can be assayed by hybridizing a chemiluminescent, acridinium ester-labeled probe to the target sequence, selectively hydrolyzing the acridinium ester present on unhybridized probe, and measuring the chemiluminescence produced from the remaining acridinium ester in a luminometer. *See, e.g.,* Arnold et al., U.S. Patent No. 5,283,174, and Nelson et al., Nonisotopic Probing, Blotting, and Sequencing, ch. 17 (Larry J. Kricka ed., 2d ed. 1995).

Hybridization Assay Probes to *T. pallidum* Ribosomal Nucleic Acid

Disclosed herein are novel hybridization assay probes useful for detecting nucleic acids (*e.g.,* nucleic acid amplification products) derived from *T. pallidum.* Hybridization is the association of two single strands of complementary nucleic acid to form a hydrogen bonded double strand. A nucleic acid sequence able to hybridize to a nucleic acid sequence sought to be detected ("target sequence") can serve as a probe for the target sequence. Hybridization may occur between complementary nucleic acid strands, including DNA/DNA, DNA/RNA, and RNA/RNA. Two single strands of deoxyribo-(DNA) or ribo-(RNA) nucleic acid, formed from nucleotides, including the bases adenine (A), cytosine (C), thymidine (T), guanine (G), uracil (U), inosine (I), and analogs thereof, may hybridize to form a double-stranded structure in which the two strands are held together by hydrogen bonds between pairs of complementary bases. Generally, A is hydrogen-bonded to T or U, while G is hydrogen-bonded to C. At any point along the hybridized strands, therefore, the classical base pairs AT or AU, TA or UA, GC or CG may be found. Thus, when a first single strand of nucleic acid contains sufficient contiguous complementary bases to a second, and those two strands are brought together under conditions that will promote their hybridization, double-stranded nucleic acid will result. Under appropriate conditions, DNA/DNA, RNA/DNA, or RNA/RNA hybrids may be formed.

The rate and extent of hybridization is influenced by a number of factors. For instance, it is implicit that if one of the two strands is wholly or partially involved in a hybrid, it will be less able to participate in the formation of a new hybrid. By designing a probe so that a substantial portion of the sequence of interest is single-stranded, the rate and extent of hybridization may be greatly increased. Also, if the target is an integrated genomic sequence it will naturally occur in a double-stranded form, as is the case with a product of PCR. These double-stranded targets are naturally inhibitory to hybridization with a probe and require denaturation prior to the hybridization step. In addition, there can be intra-molecular hybrids formed within a probe if there is sufficient self-complementarity. Regions of the nucleic acid which are known to form strong internal structures inhibitory to hybridization are typically less preferred. Examples of such structures include hairpin loops. Undesirable secondary structure in a hybridization assay probe can be avoided through careful probe design, and commercial computer programs are available to search for these types of interactions, such as the Oligo Tech® analysis software available from Oligo Therapeutics, Inc.

In some applications, such as homogenous assays, probes exhibiting at least some degree of self-complementarity may be desirable to facilitate detection of probe:target duplexes in a test sample. As discussed herein above, such probes include "molecular torches" which are designed to include distinct regions of self-complementarity referred to as the "target binding domain" and the "target closing domain." These two domains are connected by a joining region in the molecular torch and hybridize to each other under hybridization assay conditions. The joining region can be a non-nucleotide linker, such as polyethylene glycol. Molecular torches are disclosed by Becker et al., "Molecular Torches," U.S. Patent No. 6,361,945.

When exposed to denaturing conditions, the two complementary regions (which may be fully or partially complementary) of the molecular torch melt, leaving the target binding domain available for hybridization to a target sequence when the original hybridization assay conditions are restored. Molecular torches are designed so that the target binding domain favors hybridization to the target sequence over the target closing domain. The target binding domain and the target closing domain of a molecular torch include interacting labels (*e.g.*, fluorophore/quencher) positioned so that a different signal is produced when the molecular torch is self-hybridized than when the molecular torch is hybridized to a target nucleic acid, thereby permitting detection of probe:target duplexes in a test sample in the presence of unhybridized probe having viable labels associated therewith.

In accordance with the teachings of Becker et al. in U.S. Patent No. 6,361,945, hybridization assay probes of the present disclosure may be designed and constructed to include, in addition to a "target binding domain" able to distinguish between nucleic acid derived from *T. pallidum,* a "target closing domain," a "joining region" and interacting labels characteristic of a molecular torch.

Another example of a self-complementary hybridization assay probe is a "molecular beacon." Molecular beacons include nucleic acid molecules having a target complementary sequence, an affinity pair (or nucleic acid arms) holding the probe in a closed conformation in the absence of a target nucleic acid sequence, and a label pair that interacts when the probe is in a closed conformation. Hybridization of the target nucleic acid and the target complementary sequence separates the members of the affinity pair, thereby shifting the probe to an open conformation. The shift to the open conformation is detectable due to reduced interaction of the label pair, which may be, for example, a fluorophore and a quencher (*e.g*., DABCYL and EDANS). Examples of various molecular beacon configurations and applications are disclosed by Tyagi et al. in U.S. Patent No. 5,925,517. In accordance with the teachings of Tyagi *et al.,* probes according to the present disclosure may be designed and constructed to include, in addition to a "target complementary sequence" able to distinguish between nucleic acid derived from *T. pallidum,* an "affinity pair" and dual labels characteristic of a molecular beacon.

In the case of a hybridization assay, the length of the target nucleic acid sequence and, accordingly, the length of the probe sequence can be important. In some cases, there may be several sequences from a particular region, varying in location and length, which will yield probes with the desired hybridization characteristics. In other cases, one sequence may have better hybridization characteristics than another that differs merely by a single base. While it is possible for nucleic acids that are not perfectly complementary to hybridize, the longest stretch of perfectly homologous base sequence will normally primarily determine hybrid stability. While probes of different lengths and base composition may be used, preferred probes have oligonucleotides that are up to 100 bases in length, more preferably from 13 to 50 bases in length, and even more preferably from 13 to 22 bases in length.

The hybridization assay probes include a base sequence that is substantially complementary to a 23S rRNA or rDNA target sequence present in or derived from the nucleic acid of *T. pallidum* (*e.g.,* including nucleic acid amplification products). Thus, the probes are able to stably bind to a *T. pallidum* target sequence under stringent hybridization assay conditions, or under conditions used during nucleic acid amplification. As discussed above, the hybridization assay probes may have additional base sequences which do not stably bind to the target nucleic acid.

In addition to self-complementary probes, probes of the present disclosure may be designed and constructed to include an immobilized probe binding region of a capture probe, where the immobilized probe binding region is comprised of a nucleotide base sequence which can hybridize under predetermined hybridization conditions to a substantially complementary nucleotide base sequence contained in an immobilized probe joined directly or indirectly to a solid support. The immobilized probe binding region is preferably selected so that it will not stably bind under the predetermined hybridization conditions to nucleic acid from any organism which may be present in the test sample, including *T. pallidum.* Thus, a preferred nucleotide base sequence for the immobilized probe binding region of a capture probe according to the present disclosure is a homopolymer tail, such as a 3' poly dA tail matched to a 5' poly dT tail on the immobilized probe. These tails may be of any length sufficient to facilitate stable hybridization under predetermined hybridization conditions and are preferably about 30 bases in length.

The immobilized probe is preferably joined to a magnetically attractable particle which can be isolated in a reaction vessel during a purification step once the probe has had sufficient time to hybridize to target nucleic acid present in the sample. Acosta et al., in U.S. Patent No. 6,254,826 ("Assay Work Station") disclose an instrument that can be used for performing such a purification step. The capture probe is preferably designed so that the melting temperature of the capture probe:target hybrid is greater than the melting temperature of the capture probe: immobilized probe hybrid. In this way, different sets of hybridization assay conditions can be employed to facilitate hybridization of the capture probe to the target nucleic acid prior to hybridization of the capture probe to the immobilized oligonucleotide, thereby maximizing the concentration of free probe and providing favorable solution-phase hybridization kinetics. This "two-step" target capture method is discussed above and disclosed by Weisburg et al., U.S. Patent No. 6,110,678. Other target capture schemes which could be readily adapted to the present disclosure are well known in the art and include, without limitation, those disclosed by the following: Dunn et al., Methods in Enzymology, "Mapping viral mRNAs by sandwich hybridization," 65(1):468-478 (1980); Ranki et al., U.S. Patent No. 4,486,539; Stabinsky, U.S. Patent No. 4,751,177; and Becker et al., U.S. Patent No. 6,130,038.

For *T. pallidum* probes, the terms "target nucleic acid sequence," "target nucleotide sequence," "target sequence" and "target region" all refer to a nucleic acid sequence present in *T. pallidum* rRNA or rDNA, or a sequence complementary thereto, which is not present in the nucleic acid of a closely related non-*T. pallidum* species.

*T. pallidum* probes of the present disclosure comprise oligonucleotides having a target-hybridizing sequence of at least 13 nucleotides long and fully contained within a base sequence selected from the group consisting of SEQ ID NO: 19, SEQ ID NO:20, or SEQ ID NO:21, or the complements thereof, allowing for substitution of RNA and DNA equivalent bases and nucleotide analogs. The probe preferentially hybridizes under stringent hybridization conditions to a target nucleic acid derived from *T. pallidum* over nucleic acid derived from non-*T. pallidum* organisms. In some embodiments, the probe does not include any other target complementary base sequence region overlapping with or in addition to the target-hybridizing sequence of bases which is capable of forming a stable hybrid with nucleic acid derived from *T. pallidum* under the same conditions. In some embodiments, the probe is labeled with a fluorophore and a quencher, and optionally includes an internally disposed non-nucleotide linker (*e.g.,* a C9 linker).

The probes may be labeled with a detectable label or reporter group by any well-known method. For example, the probe may be labeled with a detectable moiety such as a radioisotope, antigen or chemiluminescent moiety to facilitate detection of the target sequence. Useful labels include radioisotopes as well as non-radioactive reporting groups. Isotopic labels include $^3$H, $^{35}$S, $^{32}$P, $^{125}$I, $^{57}$Co and $^{14}$C. Isotopic labels can be introduced into an oligonucleotide by techniques known in the art such as nick translation, end labeling, second strand synthesis, reverse transcription and by chemical methods. When using radiolabeled probes, hybridization can be detected by techniques such as autoradiography, scintillation counting or gamma counting. The chosen detection method depends on the particular radioisotope used for labeling.

As discussed above, non-isotopic materials can also be used for labeling and may be introduced internally between nucleotides or at an end of the oligonucleotide. Modified nucleotides can be incorporated enzymatically or chemically. Chemical modifications of the oligonucleotide may be performed during or after synthesis of the oligonucleotide using techniques known in the art. For example, through use of non-nucleotide linker groups disclosed by Arnold et al.

in U.S. Patent No. 6,031,091. Non-isotopic labels include fluorescent molecules, chemiluminescent molecules, fluorescent chemiluminescent molecules, phosphorescent molecules, electrochemiluminescent molecules, chromophores, enzymes, enzyme cofactors, enzyme substrates, dyes and haptens or other ligands. Another useful labeling technique is a base sequence that is unable to stably bind to the target nucleic acid under stringent conditions. Probes of the present disclosure can be labeled with an acridinium ester which is joined to the probe by means of a non-nucleotide linker. Acridinium ester labeling techniques are disclosed by Arnold et al. in U.S. Patent No. 5,185,439. Linking reagents are disclosed by Arnold et al. in U.S. Patent No. 6,031,091.

The selected hybridization assay probe can be contacted with a test sample suspected of containing *T. pallidum* nucleic acid. Generally, the test sample is from a source which also contains unknown organisms. After bringing the probe into contact with the test sample, which may include amplified nucleic acids derived from *T. pallidum,* the test sample can be incubated under conditions permitting preferential hybridization of the probe to a target nucleic acid derived from *T. pallidum* over nucleic acid derived from non-target organisms in the test sample.

Nucleic Acid Compositions

In another related aspect, the present disclosure features compositions comprising a nucleic acid hybrid formed between a hybridization assay probe and a target nucleic acid ("probe:target"). One use of the hybrid formed between a probe and a target nucleic acid is to provide an indication of the presence or amount of a target organism or group of organisms in a test sample.

The present disclosure also contemplates compositions including a nucleic acid hybrid formed between a capture probe and a target nucleic acid ("capture probe:target"), for example under stringent hybridization assay conditions. One use of the hybrid formed between a capture probe and a target nucleic acid is to isolate and purify the target nucleic acid in a test sample prior to amplification of a target sequence contained in the target nucleic acid or detection of the target nucleic acid in, for example, a heterogenous assay. By isolating and purifying target nucleic acid prior to amplification or detection, the opportunities for non-specific binding or amplification are advantageously reduced.

The present disclosure additionally features compositions comprising a nucleic acid hybrid formed between an amplification primer and a target nucleic acid ("primer:target") under amplification conditions. One use of the hybrid formed between a primer and a target nucleic acid is to provide an initiation site for a nucleic acid polymerase at the 3' end of the amplification primer. For example, a hybrid may form an initiation site for reverse transcriptase, DNA polymerases such as *Taq* polymerase or T4 DNA polymerase, and RNA polymerases such as T7 polymerase, SP6 polymerase, T3 polymerase and the like.

Compositions of the present disclosure include compositions for determining the presence or amount of *T. pallidum* in a test sample comprising a nucleic acid hybrid formed between a target nucleic acid derived from *T. pallidum* and a probe comprising an oligonucleotide having a target binding region, where the base sequence of the target binding region consists of the base sequence of any of the probes disclosed herein. The oligonucleotides of these compositions may include at least one additional nucleotide base sequence region which does not stably bind to nucleic acid derived from *T. pallidum* under stringent hybridization conditions.

Also contemplated by the present disclosure are compositions for immobilizing a target nucleic acid derived from *T. pallidum* present in a test sample comprising a nucleic acid hybrid formed between the target nucleic acid and a capture probe having a target binding region, where the base sequence of the target binding region is at least about 85% homologous (preferably at least about 90% homologous, more preferably at least about 95% homologous, and most preferably 100% homologous) to the base sequence of any target capture oligonucleotide disclosed herein. In a further embodiment, these compositions additionally include a nucleic acid hybrid formed between an immobilized probe binding region of the capture probe and an immobilized probe.

Assay Methods

The present disclosure contemplates various methods for determining the presence or amount of nucleic acid derived from *T. pallidum* in a test sample. One skilled in the art will understand that the exact assay conditions, probes and/or primers used will vary depending on the particular assay format used and the source of the sample.

One aspect of the present disclosure relates to a method of determining the presence or amount of *T. pallidum* in a test sample by contacting the test sample under stringent hybridization assay conditions with a hybridization assay probe capable of preferentially hybridizing under stringent hybridization conditions to nucleic acid derived from *T. pallidum* over nucleic acid derived from non-*T. pallidum* organisms present in the test sample. The probes of this method may include at least one additional base sequence region which does not stably bind to nucleic acid derived from *T. pallidum* under stringent hybridization conditions.

A further aspect of the present disclosure relates to a method for amplifying nucleic acid derived from *T. pallidum* present in a test sample by contacting the test sample under amplification conditions with one or more amplification primers, where each amplification primer comprises an oligonucleotide having a target binding region, where the base sequence of the target binding region has or substantially corresponds to the base sequence of the oligonucleotide primers presented herein. The amplification primers of this embodiment optionally include a 5' sequence which is recognized by an RNA polymerase or which enhances initiation or elongation by an RNA polymerase. When included, a T7 promoter is preferred.

In a preferred embodiment, the method for amplifying *T. pallidum-derived* nucleic acid in a test sample further includes the step of contacting the test sample under stringent hybridization assay conditions with a hybridization assay probe capable of preferentially hybridizing to an amplified *T. pallidum* target nucleic acid over nucleic acids from non-*T. pallidum* organisms present in the test sample under the stringent conditions. While the test sample optionally may be contacted with the hybridization assay probe after a sufficient period for amplification has passed, the amplification primers and hybridization assay probe may instead be added to the sample in any order, especially where the hybridization assay probe is a self-hybridizing probe, such as a molecular torch or a molecular beacon.

Certain preferred embodiments of the disclosed technique can be carried out using a real-time format, wherein synthesis of nucleic acid amplification products specific for *T. pallidum* are monitored as the amplification reaction is occurring. Molecular torches or molecular beacons can be included in the amplification reaction mixtures to permit sequence-specific detection. Molecular torches may be particularly useful for real-time detection of the target nucleic acid.

Still another aspect of the present disclosure relates to a method for immobilizing a target nucleic acid derived from a *T. pallidum* in a test sample which comprises providing to the test sample a capture probe having a target binding region and an immobilized probe binding region under a first set of hybridization conditions permitting the capture probe to stably bind the target nucleic acid, thereby forming a capture probe: target complex, and a second set of hybridization conditions permitting the capture probe to stably bind to an immobilized probe in the test sample, thereby forming an immobilized probe:capture probe:target complex. The first and second sets of hybridization conditions may be the same or different and the capture probe:target complex remains stable under the second set of hybridization conditions. The target-hybridizing sequence of bases of the capture probe can have the base sequence of SEQ ID NO:34, SEQ ID NO:35, or SEQ ID NO:36. Target capture oligonucleotides may further include a 3' immobilized probe binding region having a poly(dA) tail 30 bases in length, as given by SEQ ID NO:37, SEQ ID NO:38, or SEQ ID NO:39.

Oligonucleotide segments incorporating these target binding region sequences can be synthesized to include 2'-O-methyl substitutions (sometimes "2' methoxy" or "2'OMe") on the sugar residues of the oligonucleotide backbone.

A purifying step can follow the immobilizing step to remove one or more components of the test sample which might interfere with or prevent amplification or specific detection of a target sequence contained in the immobilized target nucleic acid. This method for immobilizing and optionally purifying a *T. pallidum-derived* nucleic may precede any of the methods described above for amplifying and/or detecting the presence of a target nucleic acid derived from *T. pallidum*. If a purifying step is included, the target nucleic acid may be indirectly eluted or separated from the immobilized probe, or directly eluted or separated from the capture probe of the immobilized probe:capture probe:target complex by altering the sample conditions prior to amplifying or detecting the target sequence.

Kits

The present disclosure also features kits useful for detecting and/or quantifying *T. pallidum.* A kit may contain, in an amount sufficient for at least one assay, any of the hybridization assay probes, capture probes and/or amplification primers of the present disclosure in a suitable packaging material as will be familiar to those having an ordinary level of skill in the art. Typically, the kits will also include instructions recorded in a tangible form (*e.g.,* contained on paper or an electronic medium) for using the packaged probes and/or primers in an amplification and/or detection assay for determining the presence or amount of *T. pallidum* in a test sample. The various kit components may be provided in a variety of forms. For example, the required enzymes, the nucleotide triphosphates, the probes and/or primers may be provided as a lyophilized reagent. Components of the lyophilized reagent may be pre-mixed before lyophilization so that when reconstituted they form a complete mixture with the proper ratio of each of the components ready for use in the assay. In addition, the kits of the present disclosure may contain a reconstitution reagent for reconstituting the lyophilized reagents. In certain preferred kits for amplifying target nucleic acid derived from *T. pallidum,* the enzymes, nucleotide triphosphates and required cofactors for the enzymes are provided as a single lyophilized reagent that, when reconstituted, forms a proper reagent for use in the present amplification methods. In these kits, a lyophilized primer reagent may also be provided. In other preferred kits, lyophilized probe reagents are provided. Typical packaging materials would include solid matrices such as glass, plastic, paper, foil, micro-particles and the like, capable of holding within fixed limits hybridization assay probes, capture probes and/or amplification primers of the present disclosure. Thus, for example, the packaging materials can include vials (*e.g.,* glass or plastic) used to contain sub-milligram (*e.g.,* picogram or nanogram) quantities of a contemplated probe or primer, or they can be microtiter plate wells to which probes or primers of the present disclosure have been operatively affixed (*i.e.,* linked so as to be capable of participating in an amplification and/or detection method of the present disclosure).

The instructions will typically indicate the reagents and/or concentrations of reagents and at least one assay method parameter which might be, for example, the relative amounts of reagents to use per amount of sample. In addition, such specifics as maintenance, time periods, temperature and buffer conditions may also be included.

As indicated above, the kits of the present disclosure may include any of the hybridization assay probes, capture probes and/or amplification primers described herein, whether provided individually or in one of the disclosed combinations described above, for use in amplifying and/or determining the presence or amount of *T. pallidum* in a test sample.

Examples are provided below to illustrate different aspects and embodiments of the disclosure. Skilled artisans will appreciate that these Examples are not intended to limit the disclosure to the specific embodiments described therein.

Examples

The disclosed technique was illustrated using a real-time Transcription Mediated Amplification (TMA) reaction format, with dual-labeled molecular torch hybridization probes being used to detect *T. pallidum* nucleic acid amplification products. All of the hybridization assay probes described below, as well as the capture probes, primers and promoter-primers, were synthesized using standard phosphoramidite chemistry and standard procedures well known in the art. *See, e.g.,* Caruthers et al., Methods in Enzymol., 154:287 (1987). Synthesis was performed using an Expedite™ 8909 Nucleic Acid Synthesizer (Applied Biosystems; Foster City, CA).

Systems developed for amplifying and detecting ribosomal nucleic acids of *T. pallidum* included probes, primers, capture oligonucleotides, and combinations thereof. Notably, a single bacterial cell contains at least about 1,000 copies of the 23S rRNA. Thus, results showing detectability of 1,000 or fewer copies of the 23S target nucleic acid would be consistent with the ability of the procedure to detect a single bacterial cell. Details are given in the following Examples.

Example 1 describes the screening of oligonucleotides in real-time TMA reactions that amplified and detected the 23s ribosomal nucleic acids of *T. pallidum*. Advantageously, *T. pallidum* nucleic acid sequences were detected without also detecting nucleic acid sequences of the closely related *T. denticola* bacterium.

**Example 1**

Screening Oligonucleotide Combinations Specific for 23s Ribosomal Nucleic Acid Detection

*In vitro* transcripts served as target nucleic acids harboring ribosomal RNA sequences in the screening procedure. DNA inserts encoding a portion of the *T. pallidum* 23S ribosomal nucleic acid sequence were ligated into a plasmid cloning vector downstream of a T7 promoter and then propagated in a bacterial host using standard laboratory procedures. Purified plasmid was linearized by restriction enzyme digestion, and then used as a template for T7 RNA polymerase to produce *T. pallidum in vitro* transcripts (IVTs), which were then quantified by spectrophotometry. The *T. pallidum* IVT included the sequence of SEQ ID NO:1. Parallel procedures were used to prepare a plasmid cloning vector harboring a DNA insert encoding a portion of the *T. denticola* 23S ribosomal nucleic acid sequence downstream of a T7 promoter. Again, the plasmid was propagated in a bacterial host, purified, linearized by restriction enzyme digestion, and used to produce *T. denticola* IVTs, which also were quantified by spectrophotometry. The *T. denticola* IVT included the sequence of SEQ ID NO:2.

Combinations of oligonucleotide primers and probes were screened for the ability to amplify and specifically detect nucleic acid sequences of *T. pallidum.* Table 1 lists the oligonucleotide combinations used in the procedure.

**Table 1**

| Oligonucleotide Screening Combinations | | | |
|---|---|---|---|
| System | Non-T7 Primer | Torch (Target-Hybridizing Sequence) [Full Sequence] | T7 Promoter Primer (Target-Hybridizing Sequence) [Full Sequence] |
| S1 | SEQ ID NO:16 | (SEQ ID NO:22) [SEQ ID NO:28] | (SEQ ID NO:6) [SEQ ID NO:10] |
| S2 | | (SEQ ID NO:23) [SEQ ID NO:29] | (SEQ ID NO:7) [SEQ ID NO:11] |
| S3 | SEQ ID NO:17 | (SEQ ID NO:24) [SEQ ID NO:30] | (SEQ ID NO:6) [SEQ ID NO:10] |
| S4 | | (SEQ ID NO:25) [SEQ ID NO:31] | (SEQ ID NO:8) [SEQ ID NO:12] |

(continued)

| | Oligonucleotide Screening Combinations | | | |
|---|---|---|---|---|
| System | Non-T7 Primer | Torch (Target-Hybridizing Sequence) [Full Sequence] | T7 Promoter Primer (Target-Hybridizing Sequence) [Full Sequence] |
| S5 | | (SEQ ID NO:26) [SEQ ID NO:32] | (SEQ ID NO:7) [SEQ ID NO:11] |
| S6 | SEQ ID NO:18 | (SEQ ID NO:25) [SEQ ID NO:31] | (SEQ ID NO:6) [SEQ ID NO:10] |
| S7 | | (SEQ ID NO:27) [SEQ ID NO:33] | (SEQ ID NO:8) [SEQ ID NO:12] |

Real-time monitoring of isothermal nucleic acid amplification reactions was carried out using a temperature-controlled MX3005p instrument (Stratagene; La Jolla, CA). Reactions were carried out in individual wells of a multiwell plate, where each well contained 65 μl of an amplification reagent that included a pH-buffered mixture of ribonucleotide triphosphates, deoxyribonucleotide triphosphates, salts and cofactors for performing TMA reactions, as will be familiar to those having an ordinary level of skill in the art. Reactions further included primers and molecular torch hybridization probes, as indicated in Table 1. Individual wells next received a 10 μl aliquot containing 1 x 10$^6$ copies of either the *T. pallidum* IVT or the *T. denticola* IVT, which served as templates or targets in the amplification reactions. All reactions were prepared in duplicate. The multiwell plate was first incubated at 60°C for 5 minutes to permit hybridization of the primers to the IVTs, and then equilibrated to about 43°C. The multiwell plate was next transferred onto an EPPENDORF THERMOMIXER® (Eppendorf North America; Westbury, NY) set at 44°C. Each reaction well next received a 25 μl aliquot of enzyme reagent containing a pH-buffered mixture of Moloney murine leukemia virus ("MMLV") reverse transcriptase and T7 RNA polymerase, also as will be familiar to those having an ordinary level of skill in the art. The plate was sealed with an adhesive cover, gently shaken for 1 minute, and then transferred into the real-time instrument set to incubate at 42°C. Fluorescent signals from the molecular torch (measured in relative fluorescence units (RFU)) were detected at regular time intervals. Threshold-based TTime values, which served as indicators of the amount of amplicon synthesized, were determined from the monitored fluorescence signals essentially according to the methods disclosed by Light et al., in U.S. Patent No. 8,615,368.

Results from the procedure, presented in Figures 1A-1F, identified several viable oligo systems that detected the *T. pallidum* target nucleic acid without detecting the *T. denticola* nucleic acid.

Example 2 describes another approach for detecting nucleic acids of *T. pallidum*. Here the target nucleic acid was amplified using a "biphasic" reaction mechanism, where linear and exponential amplification steps were separated in time. Essential features of the technique are described in U.S. Patent No. 10, 196,674. Notably, use of the biphasic amplification technique improved the sensitivity of *T. pallidum* nucleic acid detection.

**Example 2**

Amplification and Detection Using a Biphasic Amplification Procedure

A biphasic amplification procedure employed a first-phase linear amplification reaction, and a second-phase exponential amplification reaction. Primer and probe combinations for Systems S5 and S7 were used in the procedure. The first-phase amplification reaction began with a target capture step wherein pre-amplification hybrid complexes, each including a T7 promoter-primer, hybridized to its complementary *T. pallidum* target nucleic acid, were captured onto solid support magnetic beads. Target capture reactions included 400 μl volumes of a pH-buffered lithium lauryl sulfate (LLS) detergent solution, *T. pallidum* IVTs at known copy levels, one of the T7 promoter-primers (SEQ ID NO:11 for S5; and SEQ ID NO: 12 for S7), magnetic beads permitting capture or immobilization of the IVT and any hybridized T7 promoter-primer (*i.e.*, pre-amplification hybrids), and target capture oligos (SEQ ID NO:37 for both S5 and S7) that facilitated immobilization or capture of pre-amplification hybrids onto the magnetic beads. Although target capture oligonucleotides (TCOs) in this Example served to bridge bead-immobilized oligonucleotides (*i.e.*, oligo(dT)) and the IVT, other options for sequence-specifically capturing IVT directly onto beads displaying capture oligonucleotides complementary to the IVT also can be used. The target capture step permitted formation and capture of pre-amplification hybrids that formed in the reaction mixtures. Isolation and washing of magnetic beads was facilitated by use of an automated magnetic particle processor. This procedure resulted in isolated pre-amplification hybrids depleted or substantially free of unhybridized T7 promoter-primer. A first-phase linear amplification reaction was initiated by combining the isolated pre-amplification hybrids with a pH buffer, ribonucleotide triphosphates, deoxyribonucleotide triphosphates, salts, cofactors, MMLV reverse transcriptase and T7 RNA polymerase enzymes, together with non-T7 primer (SEQ ID NO:18), but no added T7 promoter primer other than that contained in the pre-amplification hybrid. Extension of the T7 primer of the pre-amplification hybrid during the first-phase reaction created a cDNA strand. RNase H activity of the MMLV reverse transcriptase digested RNA strands that served as templates for the cDNA synthesis. The non-T7 primer present in the reaction mixture hybridized to the complementary cDNA and was extended to produce a double-stranded DNA having a T7 promoter sequence. The T7 RNA polymerase of the reaction mixture then synthesized transcripts from the double-stranded template. These transcripts hybridized to the non-T7 primer that was included in the reaction mixture, which then was extended by the reverse transcriptase enzyme. Again, the RNA template strand was degraded by the RNase H activity of the MMLV reverse transcriptase. The result of these steps was accumulation in the first-phase reaction mixture of promoterless cDNA strands having the same sequence orientation or polarity as the IVT target strands.

The exponential-phase amplification reaction was initiated by adding to the first-phase reaction mixture a T7 promoter-primer (SEQ ID NO:11 for S5; and SEQ ID NO: 12 for S7) and molecular torch hybridization probe SEQ ID NO:32 for S5; and SEQ ID NO:33 for S7). In this illustrative procedure, the added T7 promoter-primer was the same as that used during the target capture step (*i.e.,* the component of the pre-amplification hybrid). Other T7 promoter-primers could have been used instead. RNA transcripts produced as amplification products during the exponential-phase amplification reactions hybridized to molecular torch hybridization probes as the reactions proceeded, and the resulting complexes were detected by fluorescence emission as a function of reaction time. General aspects of the biphasic amplification technique are disclosed by Nelson et al., in U.S. Patent No. 10,196,674.

Results from the procedure, presented in Figures 2A-2B, confirmed that both System 5 and System 7 performed well using the biphasic amplification and detection format. System 5 gave exceptionally well-shaped run curves, even at only 10 copies/ml of the IVT.

Example 3 describes one set of procedures used to demonstrate sensitivity and specificity of an example assay for detecting *T. pallidum* nucleic acid. The procedure involved combining low, variable amounts of *T. pallidum* IVT and high, constant amounts of the closely related, but non-target *T. denticola* IVT. The mixtures were subjected to target capture and biphasic amplification and detection. System S5 oligonucleotides were used in this demonstration.

## Example 3

Testing Assay Sensitivity and Specificity Using IVTs

The biphasic amplification and detection procedure of Example 2, using the primer an probe oligonucleotides of System S5, was essentially followed to demonstrate assay sensitivity and specificity. Quantities and identities of target nucleic acids served as a variable. Individual target capture reactions were spiked with aliquots of either: (1) 10 copies/ml, 30 copies/ml, 100 copies/ml, or 300 copies/ml of the *T. pallidum* IVT to establish a baseline for comparison; or (2) 0 copies/ml of *T. pallidum* IVT and $1 \times 10^6$ copies/ml of *T. denticola,* 30 copies/ml of *T. pallidum* IVT and $1 \times 10^6$ copies/ml of *T. denticola,* or 100 copies/ml of *T. pallidum* IVT and $1 \times 10^6$ copies/ml of *T. denticola.* First phase linear, and second phase exponential amplification reactions were carried out as described in the preceding Example.

Results of the procedure, shown in Figures 3A-3B, confirmed that the *T. pallidum* assay was highly specific for the *T. pallidum* target nucleic acid, and was not substantially compromised by the presence of high amounts of closely related non-target nucleic acids. Figure 3A shows well shaped run curves representing detection of *T. pallidum* amplification products, even at the level of 10 copies/ml. Thus, the assay was very sensitive. Figure 3B demonstrated that *T. pallidum* amplification products were easily detectable at 300 copies/ml or 30 copies/ml in the presence of high levels of the *T. denticola* non-target nucleic acid. Moreover, no amplification products of the *T. denticola* target nucleic acid could be detected. Thus, the assay was both highly sensitive and highly specific.

Example 4 describes procedures used to investigate specificity and sensitivity of the *T. pallidum* assay using cells instead of IVTs. A curve-fitting analysis of positivity results obtained using known numbers of whole *T. pallidum* organisms was used to determine assay sensitivity.

## Example 4

Testing Assay Sensitivity Using T. *pallidum* Cells

The real-time TMA assay of Example 3 was used to capture, amplify and detect 23S rRNA of *T. pallidum* for sensitivity testing using *T. pallidum* cells as the source of target nucleic acids. Samples were processed and amplification and detection reactions were carried out using a commercially available Panther® automated nucleic acid testing instrument (Hologic, Inc.; San Diego, CA). Serial dilutions of dark field quantified *T. pallidum* organisms at the levels indicated in Table 2 were processed in replicates of 10, and positive detection was determined by requiring a combination of a minimum fluorescent signal reading, and a maximum time at which a predetermined level of amplification was reached. More specifically, positives were determined as background-subtracted RFU (Relative Fluorescence Units) of at least 1.0, and Ttime readings of less than or equal to 53 minutes. Results of the procedure are presented in Table 2.

**Table 2**

| Whole Organism Sensitivity Testing | | |
|---|---|---|
| *T. pallidum* cells/ml | # Replicates | # Positive |
| 0.003 | 10 | 0 |
| 0.01 | 10 | 0 |
| 0.03 | 10 | 0 |
| 0.1 | 10 | 2 |
| 0.3 | 10 | 5 |
| 1 | 10 | 9 |
| 3 | 10 | 10 |

A statistical analysis of the results presented in Table 2 indicated a 50% probability of detecting as few as 0.27 organisms, and a 95% probability of detecting as few as 1.36 organisms using the disclosed assay.

Example 5 describes procedures that demonstrated how the assay of the preceding Example could be used to detect nucleic acids of *T. pallidum* without also detecting nucleic acids from a variety of other organisms.

**Example 5**

Testing Assay Specificity Using Bacterial Cells

A biphasic amplification and detection procedure essentially as described under Example 4 was followed to investigate specificity of the *T. pallidum* assay using a collection of non-target organisms as the source of amplifiable nucleic acids. Pooled sets of panels containing known quantities of the non-target organisms are identified in Table 3. As above, a combined lysis and target capture reagent included a pH buffer, lithium lauryl sulfate detergent, magnetic beads displaying a surface-immobilized oligonucleotide to aid in capturing nucleic acids. In this instance, the surface-immobilized oligonucleotide included oligo (dT), and the target capture reagent further included the target capture oligonucleotide of SEQ ID NO:37 and a T7 promoter primer that included the target-hybridizing sequence of SEQ ID NO:7 (*e.g.,* SEQ ID NO:11). Beads having captured complexes that included a target nucleic acid and a hybridized primer were washed, and then combined with an amplification reagent that included deoxyribonucleotide triphosphates, ribonucleotide triphosphates, MMLV reverse transcriptase, T7 RNA polymerase, salts and cofactors, and the non-T7 primer having the sequence of SEQ ID NO:18 to begin the linear phase amplification. After an initial incubation period, the reaction mixture was combined with an aliquot of reagent that included a molecular torch having the sequence of SEQ ID NO:32, and T7 promoter primer to initiate the exponential phase amplification reaction. In this case, the added T7 promoter was the same as that included in the target capture reagent (SEQ ID NO:11), although this was a matter of convenient choice. Different T7 promoter primers could have been used for the same purpose.

The results presented in Table 3 indicated that the *T. pallidum* assay advantageously was highly specific for detection of *T. pallidum* nucleic acids. As indicated in the table, nucleic acid sequences characteristic of *T. pallidum* were detected at a level of 100% positivity using test samples having only 30 copies/ml of the IVT. There was 0% positivity when potentially cross-reacting non-target microorganisms were present, and the *T. pallidum* target was absent. Interference testing of low titer *T. pallidum* RNA (30 copies/ml) showed 100% positivity. Assay performance was not impacted by non-target organisms.

**Table 3**

| Pool # | Organism | Final Conc. | Units | 0 c/ml TP (% Positive) | 30 c/ml TP (% Positive) |
|---|---|---|---|---|---|
| 1 | *Acinetobacter iwoffii* | 1.00E+06 | CFU/ml | 0% | 100% |
| | *\*Actinomyces israelii* | 5.00E+09 | copies rRNA/ml | | |
| | *Alcaligenes faecalis* | 1.00E+06 | CFU/ml | | |
| | *\*Atopobium vaginae* | 5.00E+09 | copies rRNA/ml | | |
| 2 | *Bacteroides fragilis* | 1.00E+06 | CFU/ml | 0% | 100% |
| | *Bifidobacterium adolescentis* | 1.00E+06 | CFU/ml | | |
| | *Campylobacter jejuni* | 1.00E+06 | CFU/ml | | |
| | *Chlamydia trachomatis* | 1.00E+05 | IFU/ml | | |
| 3 | *Candida krusei* | 1.00E+06 | CFU/ml | 0% | 100% |
| | *Candida lusitaniae* | 1.00E+06 | CFU/ml | | |
| | *Clostridium difficile* | 1.00E+06 | CFU/ml | | |
| | *Corynebacterium genitalium* | 1.00E+06 | CFU/ml | | |
| 4 | *Cryptococcus neoformans* | 1.00E+06 | CFU/ml | 0% | 100% |
| | *Eggerthella lenta* | 1.00E+06 | CFU/ml | | |
| | *Enterobacter cloacae* | 1.00E+06 | CFU/ml | | |
| | *Enterococcus faecalis* | 1.00E+06 | CFU/ml | | |
| 5 | *Escherichia coli* | 1.00E+06 | CFU/ml | 0% | 100% |
| | *Haemophilus ducreyi* | 1.00E+06 | CFU/ml | | |
| | *Klebsiella pneumoniae* | 1.00E+06 | CFU/ml | | |
| | *Listeria monocytogenes* | 1.00E+06 | CFU/ml | | |
| 6 | *Lactobacillus acidophilus* | 1.00E+06 | CFU/ml | 0% | 100% |
| | *Lactobacillus iners* | 1.00E+06 | CFU/ml | | |
| | *Lactobacillus mucosae* | 1.00E+06 | CFU/ml | | |
| | *Leptotrichia bucalis* | 1.00E+06 | CFU/ml | | |
| 7 | *\*Mobiluncus curtisii* | 5.00E+09 | copies rRNA/ml | 0% | 100% |
| | *Mycoplasma genitalium* | 1.00E+06 | CFU/ml | | |
| | *\*Mycoplasma hominis* | 5.00E+09 | copies rRNA/ml | | |
| | *Neisseria gonorrhoeae* | 1.00E+06 | CFU/ml | | |
| 8 | *Peptostreptococcus magnus* | 1.00E+06 | CFU/ml | 0% | 100% |
| | *Prevotella bivia* | 1.00E+06 | CFU/ml | | |
| | *Propionibacterium acnes* | 1.00E+06 | CFU/ml | | |
| | *Proteus vulgaris* | 1.00E+06 | CFU/ml | | |
| 9 | *Staphylococcus aureus* | 1.00E+06 | CFU/ml | 0% | 100% |
| | *Staphylococcus epidermidis* | 1.00E+06 | CFU/ml | | |
| | *Streptococcus agalactiae* | 1.00E+06 | CFU/ml | | |
| | *Streptococcus pyogenes* | 1.00E+06 | CFU/ml | | |
| 10 | *Trichomonas vaginalis* | 1.00E+05 | cells/ml | 0% | 100% |
| | *Ureaplasma Parvum* | 1.00E+06 | CFU/ml | | |
| | *Ureaplasma urealyticum* | 1.00E+06 | CFU/ml | | |

(continued)

| Pool # | Organism | Final Conc. | Units | 0 c/ml TP (% Positive) | 30 c/ml TP (% Positive) |
|---|---|---|---|---|---|
| 11 | *Herpes simplex virus I* | 1.00E+05 | TCID 50/ml | 0% | 100% |
| | *Herpes simplex virus II* | 1.00E+05 | TCID 50/ml | | |
| | *HIV* | 1.00E+06 | copies/ml | | |
| 12 | *Candida dubliniensis* | 1.00E+06 | CFU/ml | 0% | 100% |
| | *Candida albicans* | 100E+06 | CFU/ml | | |
| | *Candida glabrata* | 1.00E+06 | CFU/ml | | |
| | *Candida parapsilosis* | 1.00E+06 | CFU/ml | | |
| | *Candida tropicalis* | 1.00E+06 | CFU/ml | | |
| 13 | *Gardnerella vaginalis* | 1.00E+06 | CFU/ml | 0% | 100% |
| | *Lactobacillus crispatus* | 1.00E+06 | CFU/ml | | |
| | *Lactobacillus gasseri* | 1.00E+06 | CFU/ml | | |
| | *Lactobacillus jensenii* | 1.00E+06 | CFU/ml | | |

It is to be understood that both the foregoing general description and detailed description are exemplary and explanatory only and are not restrictive of the teachings. To the extent that any material incorporated by reference is inconsistent with the express content of this disclosure, the express content controls.

While the present disclosure has been described and shown in considerable detail with reference to certain illustrative embodiments, including various combinations and sub-combinations of features, those skilled in the art will readily appreciate other embodiments and variations and modifications thereof as encompassed within the scope of the present disclosure. Moreover, the descriptions of such embodiments, combinations, and sub-combinations is not intended to convey that the disclosure requires features or combinations of features other than those expressly recited in the claims.

**Claims**

1.  A method for determining whether a sample comprises nucleic acids of *T. pallidum,* the method comprising the steps of:

    (a) contacting the sample with an oligonucleotide primer set comprising a first primer and a second primer,

        wherein the first primer is up to 50 bases in length and comprises a target-hybridizing sequence at the 3' terminus of the first primer consisting of SEQ ID NO:6, SEQ ID NO:7 or SEQ ID NO:8, and
        wherein the second primer is up to 50 bases in length and comprises a target-hybridizing sequence at the 3' terminus of the second primer consisting of SEQ ID NO: 16, SEQ ID NO: 17 or SEQ ID NO: 18; and
        wherein at least one of the first and second primers includes a phage promoter sequence attached upstream of the target-hybridizing sequence; and

    (b) amplifying any nucleic acids of *T. pallidum* that may be present in the sample using the set of primers in an in vitro nucleic acid amplification reaction,
    whereby an amplification product is produced if the sample comprises nucleic acids of *T. pallidum,*
    (c) detecting any of the amplification product produced in step (b) using a detectably labeled oligonucleotide hybridization probe comprising:

        a target-hybridizing sequence of at least 13 contiguous bases of SEQ ID NO: 19 or the complement thereof, allowing for substitution of RNA and DNA equivalent bases; and
        a detectable label,
        wherein the oligonucleotide hybridization probe is up to 47 bases in length; and

    (d) determining from the result of step (c) whether the amplification product was produced in step (b) as an indication whether the sample comprises nucleic acids of *T. pallidum.*

2. The method of claim 1, wherein the detectably labeled hybridization probe comprises:

a target-hybridizing sequence consisting of SEQ ID NO:22, SEQ ID NO:23, SEQ ID NO:24, SEQ ID NO:25, SEQ ID NO:26 or SEQ ID NO:27, each of these sequences allowing for substitution of RNA and DNA equivalent bases; and
a detectable label,
wherein the oligonucleotide hybridization probe is up to 47 bases in length.

3. The method of any claim 1 or claim 2, wherein steps (a) and (b) take place concurrently, the amplification reaction being a real-time amplification reaction; and/or
wherein the amplification product detected in step (c) is an RNA amplicon that is opposite sense to the 23S rRNA of *T. pallidum.*

4. A reaction mixture for detecting nucleic acids of *T. pallidum* that may be present in a test sample, the reaction mixture comprising:

the test sample;
an oligonucleotide primer set comprising a first primer and a second primer,

wherein the first primer comprises a target-hybridizing sequence at the 3' terminus of the first primer consisting of SEQ ID NO:6, SEQ ID NO:7 or SEQ ID NO:8, and
wherein the second primer comprises a target-hybridizing sequence at the 3' terminus of the second primer consisting of SEQ ID NO: 16, SEQ ID NO: 17 or SEQ ID NO: 18; and
wherein the first primer optionally includes a phage promoter sequence attached upstream of the target-hybridizing sequence; and

a detectably labeled hybridization probe, said probe comprising a base sequence complementary to an amplicon produced in a nucleic acid amplification reaction performed using the oligonucleotide primer set and a template comprising the base sequence of SEQ ID NO: 1.

5. The reaction mixture of claim 4, wherein the base sequence complementary to the amplicon is, allowing for RNA and DNA equivalent base substitutions, selected from the group consisting of SEQ ID NO:22, SEQ ID NO:23, SEQ ID NO:24, SEQ ID NO:25, SEQ ID NO:26, and SEQ ID NO:27; or

wherein the first primer is up to 50 bases in length and comprises a promoter sequence upstream of the sequence complementary to at least 18 contiguous bases of SEQ ID NO: 3; preferably,
wherein the promoter sequence is a T7 promoter sequence.

6. The reaction mixture of claim 4 or claim 5,

wherein the amplicon is an RNA amplicon with polarity opposite to 23S rRNA of *T. pallidum,* so that the RNA amplicon is complementary to 23S rRNA of *T. pallidum;* preferably,
wherein the reaction mixture comprises the RNA amplicon hybridized to the molecular torch hybridization probe.

7. A kit of reagents for detecting nucleic acids of *T. pallidum,* comprising:

a set of oligonucleotide primers,

wherein a first primer of the set comprises a target-hybridizing sequence at the 3' terminus of the first primer consisting of SEQ ID NO:6, SEQ ID NO:7 or SEQ ID NO:8, and
wherein a second primer of the set comprises a target-hybridizing sequence at the 3' terminus of the second primer consisting of SEQ ID NO: 16, SEQ ID NO: 17 or SEQ ID NO: 18;

a molecular torch hybridization probe, up to 50 bases in length, comprising a target-hybridizing sequence of at least 13 contiguous bases of SEQ ID NO: 19 or the complement thereof, and further comprising each of a fluorophore moiety, a quencher moiety, a non-nucleotide linker, and at least one nucleotide analog comprising a ribofuranosyl moiety with a 2' -O-methyl substitution; and
one or more reagents for performing an *in vitro* nucleic acid amplification reaction using the set of primers.

8. The kit of claim 7, wherein the target-hybridizing sequence of at least 13 contiguous bases of SEQ ID NO: 19 or the complement thereof is a target-hybridizing sequence of 13 to 22 contiguous bases of SEQ ID NO:21 or the complement thereof; and/or

wherein the target-hybridizing sequence of the molecular torch hybridization probe is selected from the group consisting of SEQ ID NO:26 and SEQ ID NO:27; and/or
wherein the target-hybridizing sequence of the molecular torch hybridization probe is selected from the group consisting of SEQ ID NO:22, SEQ ID NO:23, SEQ ID NO:24, SEQ ID NO:25, SEQ ID NO:26, and SEQ ID NO:27; and/or
wherein each of the first and second primers is up to 50 bases in length.

9. The kit of claim 7 or claim 8, wherein a phage promoter sequence is attached upstream of the target-hybridizing sequence of the first primer.

10. The kit of claim 9, wherein the phage promoter sequence is a T7 promoter sequence.

11. The kit of any one of claims 7 to 10, wherein the first primer is a promoter-primer selected from the group consisting of SEQ ID NO: 10, SEQ ID NO: 11 and SEQ ID NO: 12; and/or
wherein the one or more reagents comprise each of a reverse transcriptase, and a T7 RNA polymerase.

**Patentansprüche**

1. Verfahren zum Bestimmen, ob eine Probe Nukleinsäuren von *T. pallidum* umfasst, wobei das Verfahren die folgenden Schritte umfasst:

(a) Inkontaktbringen der Probe mit einem Oligonukleotid-Primersatz, umfassend einen ersten Primer und einen zweiten Primer,

wobei der erste Primer bis zu 50 Basen lang ist und eine Zielhybridisierungssequenz am 3'-Terminus des ersten Primers umfasst, bestehend aus SEQ ID NO:6, SEQ ID NO:7 oder SEQ ID NO:8, und
wobei der zweite Primer bis zu 50 Basen lang ist und eine Zielhybridisierungssequenz am 3'-Terminus des zweiten Primers umfasst, bestehend aus SEQ ID NO:16, SEQ ID NO:17 oder SEQ ID NO:18; und
wobei mindestens einer der ersten und zweiten Primer eine Phagenpromotorsequenz beinhaltet, die der Zielhybridisierungssequenz vorgelagert angebracht ist; und

(b) Amplifizieren aller Nukleinsäuren von *T. pallidum,* die in der Probe vorhanden sein können, unter Verwendung des Satzes von Primern in einer in-vitro-Nukleinsäureamplifikationsreaktion,
wobei ein Amplifikationsprodukt produziert wird, wenn die Probe Nukleinsäuren von *T. pallidum* umfasst,
(c) Nachweisen eines beliebigen Amplifikationsprodukts, das in Schritt (b) unter Verwendung einer nachweisbar markierten Oligonukleotid-Hybridisierungssonde produziert wurde, umfassend:

eine Zielhybridisierungssequenz von mindestens 13 aufeinanderfolgenden Basen von SEQ ID NO:19 oder dem Komplement davon, wobei Substitution von RNA- und DNA-äquivalenten Basen zulässig ist; und
eine nachweisbare Markierung,
wobei die Oligonukleotid-Hybridisierungssonde bis zu 47 Basen lang ist; und

(d) Bestimmen anhand des Ergebnisses von Schritt (c), ob das Amplifikationsprodukt in Schritt (b) produziert wurde, als Hinweis, ob die Probe Nukleinsäuren von *T. pallidum* umfasst.

2. Verfahren nach Anspruch 1, wobei die nachweisbar markierte Hybridisierungssonde umfasst:

eine Zielhybridisierungssequenz, bestehend aus SEQ ID NO:22, SEQ ID NO:23, SEQ ID NO:24, SEQ ID NO:25, SEQ ID NO:26 oder SEQ ID NO:27, wobei jede dieser Sequenzen die Substitution von RNA- und DNA-äquivalenten Basen zulässt; und

eine nachweisbare Markierung,
wobei die Oligonukleotid-Hybridisierungssonde bis zu 47 Basen lang ist.

3. Verfahren nach Anspruch 1 oder Anspruch 2, wobei die Schritte (a) und (b) gleichzeitig stattfinden, wobei die Amplifikationsreaktion eine Echtzeit-Amplifikationsreaktion ist; und/oder
wobei das in Schritt (c) nachgewiesene Amplifikationsprodukt ein RNA-Amplikon ist, das gegensinnig zur 23S-rRNA von *T. pallidum* ist.

4. Reaktionsgemisch zum Nachweisen von Nukleinsäuren von *T. pallidum,* die in einer Testprobe vorhanden sein können, wobei das Reaktionsgemisch umfasst:

die Testprobe;
einen Oligonukleotid-Primersatz, umfassend einen ersten Primer und einen zweiten Primer,

wobei der erste Primer eine Zielhybridisierungssequenz am 3'-Terminus des ersten Primers umfasst, bestehend aus SEQ ID NO:6, SEQ ID NO:7 oder SEQ ID NO:8, und
wobei der zweite Primer eine Zielhybridisierungssequenz am 3'-Terminus des zweiten Primers umfasst, bestehend aus SEQ ID NO:16, SEQ ID NO:17 oder SEQ ID NO:18; und
wobei der erste Primer optional eine Phagenpromotorsequenz beinhaltet, die der Zielhybridisierungssequenz vorgelagert angebracht ist; und

eine nachweisbar markierte Hybridisierungssonde, wobei die genannte Sonde eine Basensequenz komplementär zu einem Amplikon umfasst, das in einer Nukleinsäureamplifikationsreaktion produziert wurde, die unter Verwendung des Oligonukleotid-Primersatzes und einer Matrize durchgeführt wurde, umfassend die Basensequenz von SEQ ID NO:1.

5. Reaktionsgemisch nach Anspruch 4, wobei die Basensequenz komplementär zum Amplikon ist, wobei RNA- und DNA-äquivalente Basensubstitutionen zulässig sind, ausgewählt aus der Gruppe bestehend aus SEQ ID NO:22, SEQ ID NO:23, SEQ ID NO:24, SEQ ID NO:25, SEQ ID NO:26 und SEQ ID NO:27; oder

wobei der erste Primer bis zu 50 Basen lang ist und eine Promotorsequenz vorgelagert der Sequenz komplementär zu mindestens 18 aufeinanderfolgenden Basen von SEQ ID NO:3 umfasst; vorzugsweise
wobei die Promotorsequenz eine T7-Promotorsequenz ist.

6. Reaktionsgemisch nach Anspruch 4 oder Anspruch 5,

wobei das Amplikon ein RNA-Amplikon mit einer Polarität entgegengesetzt zu 23S rRNA von *T. pallidum* ist, so dass das RNA-Amplikon komplementär zu 23S rRNA von *T. pallidum* ist; vorzugsweise
wobei das Reaktionsgemisch das RNA-Amplikon umfasst, das mit der molekularen Torch-Hybridisierungssonde hybridisiert ist.

7. Kit von Reagenzien zum Nachweisen von Nukleinsäuren von *T. pallidum,* umfassend:

einen Satz von Oligonukleotid-Primern,

wobei ein erster Primer des Satzes eine Zielhybridisierungssequenz am 3'-Terminus des ersten Primers umfasst, bestehend aus SEQ ID NO:6, SEQ ID NO:7 oder SEQ ID NO:8, und
wobei ein zweiter Primer des Satzes eine Zielhybridisierungssequenz am 3'-Terminus des zweiten Primers umfasst, bestehend aus SEQ ID NO:16, SEQ ID NO:17 oder SEQ ID NO:18;

eine molekulare Torch-Hybridisierungssonde mit einer Länge von bis zu 50 Basen, umfassend eine Zielhybridisierungssequenz von mindestens 13 aufeinanderfolgenden Basen von SEQ ID NO:19 oder das Komplement davon, und ferner umfassend jeweils eine Fluorophor-Einheit, eine Quencher-Einheit, einen Nicht-Nukleotid-Linker und mindestens ein Nukleotid-Analogon, umfassend eine Ribofuranosyl-Einheit mit einer 2'-O-Methyl-Substitution; und
ein oder mehrere Reagenzien zum Durchführen einer *In-vitro*-Nukleinsäureamplifikationsreaktion unter Verwendung des Satzes von Primern.

8. Kit nach Anspruch 7, wobei die Zielhybridisierungssequenz von mindestens 13 aufeinanderfolgenden Basen von SEQ ID NO:19 oder dem Komplement davon eine Zielhybridisierungssequenz von 13 bis 22 aufeinanderfolgenden Basen von SEQ ID NO:21 oder dem Komplement davon ist; und/oder

wobei die Zielhybridisierungssequenz der molekularen Torch-Hybridisierungssonde ausgewählt ist aus der Gruppe bestehend aus SEQ ID NO:26 und SEQ ID NO:27; und/oder
wobei die Zielhybridisierungssequenz der molekularen Torch-Hybridisierungssonde ausgewählt ist aus der Gruppe bestehend aus SEQ ID NO:22, SEQ ID NO:23, SEQ ID NO:24, SEQ ID NO:25, SEQ ID NO:26 und SEQ ID NO:27; und/oder
wobei jeder der ersten und zweiten Primer bis zu 50 Basen lang ist.

9. Kit nach Anspruch 7 oder 8, wobei eine Phagenpromotorsequenz der Zielhybridisierungssequenz des ersten Primers vorgelagert angebracht ist.

10. Kit nach Anspruch 9, wobei die Phagenpromotorsequenz eine T7-Promotorsequenz ist.

11. Kit nach einem der Ansprüche 7 bis 10, wobei der erste Primer ein Promotorprimer ist, der ausgewählt ist aus der Gruppe bestehend aus SEQ ID NO:10, SEQ ID NO:11 und SEQ ID NO:12; und/oder
wobei das eine oder mehrere Reagenzien jeweils eine reverse Transkriptase und eine T7-RNA-Polymerase umfassen.

**Revendications**

1. Procédé pour déterminer si un échantillon comprend des acides nucléiques de *T. pallidum,* le procédé comprenant les étapes de :

(a) mise en contact de l'échantillon avec un jeu d'amorces oligonucléotidiques comprenant une première amorce et une deuxième amorce,

dans lequel la première amorce mesure jusqu'à 50 bases de longueur et comprend, à l'extrémité 3' de la première amorce, une séquence d'hybridation cible constituée de SEQ ID NO:6, SEQ ID NO:7 ou SEQ ID NO:8, et
dans lequel la deuxième amorce mesure jusqu'à 50 bases de longueur et comprend, à l'extrémité 3' de la deuxième amorce, une séquence d'hybridation cible constituée de SEQ ID NO:16, SEQ ID NO:17 ou SEQ ID NO:18 ; et
dans lequel au moins l'une parmi les première et deuxième amorces inclut une séquence promotrice de phage fixée en amont de la séquence d'hybridation cible ; et

(b) amplification de tout acide nucléique de *T. pallidum* qui peut être éventuellement présent dans l'échantillon à l'aide du jeu d'amorces au cours d'une réaction d'amplification d'acides nucléiques in vitro,
par laquelle un produit d'amplification est produit si l'échantillon comprend des acides nucléiques de *T. pallidum,*
(c) détection de l'un quelconque des produits d'amplification produits à l'étape (b) à l'aide d'une sonde d'hybridation oligonucléotidique marquée de manière détectable comprenant :

une séquence d'hybridation cible d'au moins 13 bases contiguës de SEQ ID NO:19 ou de son complément, permettant la substitution de bases équivalentes d'ARN et d'ADN ; et
un marqueur détectable,
dans lequel la sonde d'hybridation oligonucléotidique mesure jusqu'à 47 bases de longueur ; et

(d) détermination, à partir du résultat de l'étape (c), si le produit d'amplification a été produit à l'étape (b) à titre d'indication que l'échantillon comprend ou non des acides nucléiques de *T. pallidum.*

2. Procédé de la revendication 1, dans lequel la sonde d'hybridation marquée de manière détectable comprend :

une séquence d'hybridation cible constituée de SEQ ID NO:22, SEQ ID NO:23, SEQ ID NO:24, SEQ ID NO:25, SEQ ID NO:26 ou SEQ ID NO:27, chacune de ces séquences permettant la substitution de bases équivalentes d'ARN et d'ADN ; et

un marqueur détectable,
dans lequel la sonde d'hybridation oligonucléotidique mesure jusqu'à 47 bases de longueur.

3. Procédé de l'une quelconque de la revendication 1 ou de la revendication 2, dans lequel les étapes (a) et (b) ont lieu simultanément, la réaction d'amplification étant une réaction d'amplification en temps réel ; et/ou
dans lequel le produit d'amplification détecté à l'étape (c) est un amplicon d'ARN de sens opposé à celui de l'ARNr 23S de *T. pallidum.*

4. Mélange réactionnel pour détecter des acides nucléiques de *T. pallidum* qui sont éventuellement présents dans un échantillon d'essai, le mélange réactionnel comprenant :

l'échantillon d'essai ;
un jeu d'amorces oligonucléotidiques comprenant une première amorce et une deuxième amorce,

dans lequel la première amorce comprend, à l'extrémité 3' de la première amorce, une séquence d'hybridation cible constituée de SEQ ID NO:6, SEQ ID NO:7 ou SEQ ID NO:8, et
dans lequel la deuxième amorce comprend, à l'extrémité 3' de la deuxième amorce, une séquence d'hybridation cible constituée de SEQ ID NO:16, SEQ ID NO:17 ou SEQ ID NO:18 ; et
dans lequel la première amorce inclut éventuellement une séquence promotrice de phage fixée en amont de la séquence d'hybridation cible ; et

une sonde d'hybridation marquée de manière détectable, ladite sonde comprenant une séquence de bases complémentaire à un amplicon produit lors d'une réaction d'amplification d'acides nucléiques réalisée à l'aide du jeu d'amorces oligonucléotidiques et d'un gabarit comprenant la séquence de bases SEQ ID NO:1.

5. Mélange réactionnel de la revendication 4, dans lequel la séquence de bases complémentaire à l'amplicon, permettant la substitution de bases équivalentes d'ARN et d'ADN, est sélectionnée parmi le groupe constitué de SEQ ID NO:22, SEQ ID NO:23, SEQ ID NO:24, SEQ ID NO:25, SEQ ID NO:26 et SEQ ID NO:27 ; ou

dans lequel la première amorce mesure jusqu'à 50 bases de longueur et comprend une séquence promotrice en amont de la séquence complémentaire d'au moins 18 bases contiguës de SEQ ID NO:3 ; de préférence,
dans lequel la séquence promotrice est une séquence promotrice T7.

6. Mélange réactionnel de la revendication 4 ou de la revendication 5,

dans lequel l'amplicon est un amplicon d'ARN avec une polarité opposée à celle de l'ARNr 23S de *T. pallidum,* de sorte que l'amplicon d'ARN est complémentaire à l'ARNr 23S de *T. pallidum* ; de préférence,
dans lequel le mélange réactionnel comprend l'amplicon d'ARN hybridé à la sonde d'hybridation de type sonde fluorescente (molecular torch).

7. Kit de réactifs pour détecter des acides nucléiques de *T. pallidum,* comprenant :

un jeu d'amorces oligonucléotidiques,

dans lequel une première amorce du jeu comprend, à l'extrémité 3' de la première amorce, une séquence d'hybridation cible constituée de SEQ ID NO:6, SEQ ID NO:7 ou SEQ ID NO:8, et
dans lequel une deuxième amorce du jeu comprend, à l'extrémité 3' de la deuxième amorce, une séquence d'hybridation cible constituée de SEQ ID NO:16, SEQ ID NO:17 ou SEQ ID NO:18 ;

une sonde d'hybridation de type sonde fluorescente (molecular torch), jusqu'à 50 bases de longueur, comprenant une séquence d'hybridation cible d'au moins 13 bases contiguës de SEQ ID NO:19 ou de son complément, et comprenant en outre chacun parmi un fragment fluorophore, un fragment quencheur, un lien non nucléotidique, et au moins un analogue nucléotidique comprenant un fragment ribofuranosyle avec une substitution en 2'-O-méthyle ; et
un ou plusieurs réactifs pour réaliser une réaction d'amplification d'acides nucléiques *in vitro* à l'aide du jeu d'amorces.

8. Kit de la revendication 7, dans lequel la séquence d'hybridation cible d'au moins 13 bases contiguës de SEQ ID NO:19 ou de son complément est une séquence d'hybridation cible de 13 à 22 bases contiguës de SEQ ID NO:21 ou de son complément ; et/ou

dans lequel la séquence d'hybridation cible de la sonde d'hybridation de type sonde fluorescente (molecular torch) est sélectionnée parmi le groupe constitué de SEQ ID NO:26 et SEQ ID NO:27 ; et/ou
dans lequel la séquence d'hybridation cible de la sonde d'hybridation de type sonde fluorescente (molecular torch) est sélectionnée parmi le groupe constitué de SEQ ID NO:22, SEQ ID NO:23, SEQ ID NO:24, SEQ ID NO:25, SEQ ID NO:26 et SEQ ID NO:27 ; et/ou
dans lequel chacune des première et deuxième amorces mesure jusqu'à 50 bases de longueur.

9. Kit de la revendication 7 ou de la revendication 8, dans lequel une séquence promotrice de phage est fixée en amont de la séquence d'hybridation cible de la première amorce.

10. Kit de la revendication 9, dans lequel la séquence promotrice de phage est une séquence promotrice T7.

11. Kit de l'une quelconque des revendications 7 à 10, dans lequel la première amorce est une amorce promotrice sélectionnée parmi le groupe constitué de SEQ ID NO:10, SEQ ID NO:11 et SEQ ID NO:12 ; et/ou
dans lequel lesdits un ou plusieurs réactifs comprennent chacun parmi les éléments : une transcriptase inverse et une ARN polymérase T7.

FIG. 1A

FIG. 1B

FIG. 1C

FIG. 1D

FIG. 1E

FIG. 1F

FIG. 2A

FIG. 2B

FIG. 3A

FIG. 3B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62891181
- WO 2006025672 A
- US 5925517 A, Tyagi
- US 6361945 B, Becker
- WO 9532305 A
- US 5378825 A
- WO 9313121 A
- US 5585481 A
- US 5118801 A
- US 5312728 A
- US 6835542 B
- US 6849412 B
- US 5283174 A
- US 5656207 A
- US 5658737 A
- US 5639604 A
- US 5547842 A
- US 4581333 A
- EP 0747706 A
- US 5399491 A
- US 5554516 A
- US 5437990 A
- US 5130238 A
- US 4868105 A
- US 5124246 A
- US 4786600 A
- US 4683195 A
- US 4683202 A
- US 4800159 A
- EP 0320308 A
- US 7282328 B
- US 5422252 A
- WO 8801302 A
- WO 8810315 A, Gingeras
- WO 9403472 A, McDonough
- WO 9503430 A, Ryder
- US 4851330 A, Kohne
- US 5216143 A, Hogan
- US 5840488 A, Hogan
- US 5449769 A
- US 5811538 A
- US 6031091 A, Arnold
- US 6087133 A, Dattagupta
- US 5185439 A, Arnold
- US 5958700 A, Nadeau
- US 5866336 A, Nazarenko
- US 6066483 A, Riggs
- US 5480784 A, Kacian
- US 4486539 A, Ranki
- US 4563419 A
- US 4751177 A, Stabinsky
- US 6335166 B, Ammann
- US 6110678 A, Weisburg
- US 5648124 A, Sutor
- US 6254826 B, Acosta
- US 6197563 B, Erlich
- US 5554517 A, Davey
- US 5427930 A, Birkenmeyer
- US 5686272 A, Marshall
- US 5712124 A, Walker
- US 6410278 B, Notomi
- US 6214587 B, Dattagupta
- US 5387510 A, Wu
- US 6054279 A
- US 6130038 A, Becker
- US 8615368 B, Light
- US 10196674 B

**Non-patent literature cited in the description**

- **HENAO-MARTINEZ et al.** *Nuerol Clin Pract.*, 2014, vol. 4 (2), 114-122
- **FRASER.** *Science*, 1998, vol. 281, 375-388
- **GOLDEN M. et al.** *J Clin Mircobiol*, 2029, vol. 57 (8), 1-7
- **CENTURION-LARA A. et al.** *J Clin Microbiol*, 1997, vol. 35 (6), 1348-1352
- **GULTOM, D. A. et al.** *Med J Indonesia*, 2017, vol. 26 (2), 90-96
- The Biochemistry of the Nucleic Acids. 1992, 5-36
- **VESTER ; WENGEL.** *Biochemistry*, 2004, vol. 43 (42), 13233-41
- **SAMBROOK et al.** Molecular Cloning, A Laboratory Manual. Cold Spring Harbor Laboratory Press, 1989
- **R. L. P. ADAMS et al.** The Biochemistry of the Nucleic Acids. 1992
- **J. SAMBROOK et al.** MOLECULAR CLONING: A LABORATORY MANUAL. 1989
- **SOUTHERN, E.M.** *J. Mol. Biol.*, 1975, vol. 98, 503

- **CARUTHERS et al.** Chemical Synthesis of Deoxynucleotides by the Phosphoramidite Method. *Methods Enzymol.*, 1987, vol. 154, 287
- **BARONE et al.** In Situ Activation of bis-dialkylaminephosphines -- a New Method for Synthesizing Deoxyoligonucleotides on Polymer Supports. *Nucleic Acids Res*, 1984, vol. 12 (10), 4051
- **WILK et al.** Backbone-Modified Oligonucleotides Containing a Butanediol-1,3 Moiety as a 'Vicarious Segment' for the Deoxyribosyl Moiety -- Synthesis and Enzyme Studies. *Nucleic Acids Res.*, 1990, vol. 18 (8), 2065
- **WALKER et al.** Strand Displacement Amplification -- an Isothermal, In Vitro DNA Amplification Technique. *Nucleic Acids Res.*, 1992, vol. 20 (7), 1691-1696
- **FAHY et al.** Self-sustained Sequence Replication (3SR): An Isothermal Transcription-Based Amplification System Alternative to PCR. *PCR Methods and Applications*, 1991, vol. 1, 25-33
- **HELEN H. LEE et al.** *Nucleic Acid Amplification Technologies: Application to Disease Diagnosis*, 1997
- **NELSON et al.** Nonisotopic Probing, Blotting, and Sequencing. 1995
- **DUNN et al.** Mapping viral mRNAs by sandwich hybridization. *Methods in Enzymology*, 1980, vol. 65 (1), 468-478
- **CARUTHERS et al.** *Methods in Enzymol.*, 1987, vol. 154, 287